# EUROPEAN PATENT APPLICATION

(11) **EP 2 735 571 A2**
(43) Date of publication of application: **28.05.2014**
(21) Application number: 11821186.1
(22) Date of filing: 20.10.2011
(51) Int. Cl.: C07K 16/30, C12N 5/10, C12N 15/02, C12Q 1/02, G01N 33/574, G01N 33/577, C12P 21/08

(54) **ANTIBODY FOR COLORECTAL CANCER MARKER**

(30) Priority: 01.09.2010 JP 2010195926
(71) Applicant: Bio Matrix Research, Inc., Chiba 270-0101 (JP); National Cancer Center, Tokyo 104-0045 (JP)
(72) Inventor: SATOFUKA, Hiroyuki, Nagareyama-shi Chiba 270-0101 (JP); OKABE, Youko, Nagareyama-shi Chiba 270-0101 (JP); MATSUMURA, Yasuhiro, Kashiwa-shi Chiba 277-8577 (JP); YASUNAGA, Masahiro, Kashiwa-shi Chiba 277-8577 (JP)
(74) Representative: Woods, Geoffrey Corlett
(86) International application number: PCT/IB2011/002506
(87) International publication number: WO 2012/028958

(57) **Abstract**

The purpose of the present invention is to provide a novel monoclonal antibody which binds to SLC6A6 or an extracellular domain thereof. The present invention relates to a monoclonal antibody which recognizes native SLC6A6 or a polypeptide of an extracellular domain of SLC6A6.

## Description

### TECHNICAL FIELD

The present invention relates to a novel monoclonal antibody which binds to SLC6A6 or an extracellular domain thereof, a hybridoma cell which produces the antibody and a cancer test kit using the antibody.

### BACKGROUND ART

Cancers rank among the highest causes of death in the world, and among them, colorectal cancer ranks among the highest mortality rates of cancers. Recently, the number of patients with colorectal cancer has rapidly increased in Japan, and about 60,000 persons are affected with colorectal cancer in a year. Regarding the organ-specific number of deaths, colorectal cancer shows the third highest number after stomach cancer and lung cancer. It is known that in the case of colorectal cancer, unlike other cancers, when it is an early stage cancer, it can be completely cured nearly 100% by surgery. Therefore, colorectal cancer is targeted for early stage cancer screening, and many test methods have been devised.

Currently, as a less demanding primary screening method for colorectal cancer, the fecal occult blood test is in widespread use. Using this technique, existence of human hemoglobin is examined to diagnose presence or absence of intestinal bleeding and to indirectly predict incidence of colorectal cancer.

The fecal occult blood test is widely utilized because it is a simple test, but there is a question about utility of the test. For example, a positive result in a test utilizing Hemoccult II (Astellas Pharma Inc.) requires 20 mg per day of bleeding in the large bowel. However, in an actual colorectal cancer patient, the amount of bleeding is thought to be 10 mg or less. As a result, the sensitivity of the fecal occult blood test is approximately 26%, and there have been reports that only about 1/4 of actual colorectal cancer patients can be found and the remaining 3/4 of the patients are overlooked (Non-Patent Document 1). Further, only 8.3% of all the positives actually had colorectal cancer and many false positives were included.

Other than this, as a less demanding primary screening method for colorectal cancer, the development of a method for genetic diagnosis in which nucleic acids are directly extracted from feces and genetic mutations are examined has been attempted. However, most of nucleic acids contained in feces are derived from various bacteria and normal cells, and the amount of nucleic acids derived from cancer cells is very small (about 0.05%). For this reason, it is difficult to detect mutations in cancer cell-derived genes and subtle changes in expression patterns, and therefore it is difficult to realize practical use of genetic diagnosis.

In order to improve accuracy of cancer detection, methods for efficiently collecting cancer cells from feces have been developed. As a typical technique, there is a method for collecting living cancer cells, in which all the steps from suspension of feces to collection of cells are conducted at room temperature (Patent Document 1).

According to this method, collection of living cancer cells contained in feces is simplified. However, since the Ber-EP4 antibody used in this method recognizes an epithelial cell adhesion molecule (EpCAM) which is widely expressed in epithelial cells (Non-Patent Document 2), there is a possibility that normal glandular epithelial cells of large bowel and the like contained in feces may be mixed in a cell population obtained. For this reason, in order to provide cancer diagnosis, it is required to analyze collected cells according to another test method by which cancer-specific characteristics can be detected.

In general, in order to specifically detect a specific cell, a technique of using a molecule which is specifically expressed in the cell and an antibody or aptamer is employed. Therefore, the present inventors narrowed down genes whose expression in cancer cells is enhanced, and as a marker molecule for specifically detecting cancer cells, focused attention on SLC6A6 (solute carrier family 6 (neurotransmitter transporter, taurine), member 6).

SLC6A6 is a 12-transmembrane protein consisting of 620 amino acids, and has been registered as NCBI (National Center for Biotechnology Information) Reference Sequences [RefSeq] ID: NM_003043 and NP_003034.2 (SEQ ID NO: 1: nucleotide sequence, SEQ ID NO: 2: amino acid sequence). SLC6A6 is involved in uptake of taurine into cells and cotransports taurine together with sodium ion and chloride ion. As splice variants of SLC6A6, there are the protein consisting of the amino acids from the N-terminus to position 200 and the gene thereof (SEQ ID NOs: 3 and 4), the protein consisting of the amino acids from the N-terminus to position 359 and the gene thereof (SEQ ID NOs: 5 and 6), etc.

It is disclosed that the SLC6A6 gene is one of genes whose expression level is higher in colon cancer tissue when compared to normal tissue (Patent Document 2). In Patent Document 2, 30 or more genes including slc6a6, which show a difference of the expression level between colorectal cancer tissue and normal tissue, have been identified by DNA microarray using 10 types of primary colon tumors and 10 types of normal colons, and it is suggested that all the disclosed genes can be applied to antibodies, methods for detecting a polypeptide using an antibody, methods for diagnosing a cancer, and pharmaceutical compositions comprising an antibody. However, there is no example of preparation of an antibody against slc6a6, and appropriateness at the protein level, practical utility and feasibility for diagnosis or treatment purposes have not been demonstrated. Further, Patent Document 2 describes detailed results of analysis of expression patterns using the quantitative PCR method, but the expression ratio varies and there is a case where higher expression is shown in normal tissue when compared to colon cancer tissue (Table 7). Further, the primers used for quantitative PCR (Table 6) are not included in the coding region of the protein. Since there are a plurality of splice variants of SLC6A6, even if the region amplified by the primers is used for the measurement, the expression level of SLC6A6 protein may not be precisely reflected thereby. Therefore, it is required to detect a target molecule not at the gene level but at the protein level, and for this purpose, a monoclonal antibody which recognizes a specific amino acid sequence and three-dimensional structure is essential. Further, utility for testing at the protein level must be examined in detail, considering the existence of variants.

As antibodies against SLC6A6, HPA015028 (ATLAS) and sc-166640 (SantaCruz) are publicly known.

sc-166640 is a monoclonal antibody and binds to the region of amino acid residues 397-424 of SLC6A6. Since this region extends from the transmembrane region into the inside of the cell, it is impossible to detect living cancer cells using this antibody. Further, when conducting immunostaining, SLC6A6 protein is denatured by immobilization of cells and permeabilization of cell membranes, and therefore the detection sensitivity of the antibody is reduced. Moreover, there is a problem that it is impossible to detect variants of SLC6A6 in which a portion starting at position 201 or 360 from the N-terminus is deleted.

HPA015028 is a polyclonal antibody. An extracellular domain of SLC6A6 consisting of amino acid residues 145-213 is expressed in E. coli and purified, a rabbit is immunized with the purified polypeptide obtained, and then affinity purification is conducted, thereby obtaining this antibody.

However, it is generally known that a membrane protein forms a characteristic three-dimensional structure on the cell membrane in order to function on the membrane, and that even if a portion of the extracellular domain is prepared, the inherent three-dimensional structure is not formed. Therefore, when a polyclonal antibody is prepared using a portion of such a membrane protein as an antigen, there are problems that an antibody that reacts with an inherent three-dimensional structure is obtained at a low rate and that the antibody titer is low.

In order to utilize an antibody as a tool for detection of target proteins or screening for diseases, it is essential to realize continuous and homogeneous production and supply, and therefore a monoclonal antibody is desired. In general, in the case of a membrane protein such as SLC6A6, particularly a multi-transmembrane protein, it is difficult to solubilize it and an extracellular domain thereof is small, and for these reasons and the like, it is difficult to prepare antibodies. Under present circumstances, even if a polyclonal antibody is successfully obtained from peripheral blood or the like of an immunized animal, the probability of successfully isolating antibody-producing cells is very low.

For the reasons described above, these conventional antibodies are not sufficient as antibodies for detecting SLC6A6.

### PRIOR ART DOCUMENTS

### PATENT DOCUMENTS

Patent Document 1: Japanese Laid-Open Patent Publication No. 2005-46065
Patent Document 2: Japanese National-phase PCT Laid-Open Patent Publication No. 2006-515318

### NON-PATENT DOCUMENTS

Non-Patent Document 1: Jama, Vol. 269, 1262-7, 1993
Non-Patent Document 2: J. Cell Biol. Vol. 125, 437-46, 1994

### SUMMARY OF THE INVENTION

### PROBLEMS TO BE SOLVED BY THE INVENTION

Therefore, a monoclonal antibody, which can detect SLC6A6 and can be provided as a test tool for cancer with good stability and reproducibility, and a simple and high-precision test method for cancer diagnosis using the antibody are desired.

Accordingly, the purpose of the present invention is to provide a monoclonal antibody which binds to an extracellular domain of SLC6A6 in a sample, and a hybridoma which produces the monoclonal antibody, and to establish a method for detecting a cancer using the antibody.

### MEANS FOR SOLVING THE PROBLEMS

The present inventors diligently made researches in order to solve the above-described problems, and succeeded in preparing a monoclonal antibody which recognizes an extracellular domain of SLC6A6, amino acid residues 143-216, and developing a kit for diagnosing a cancer using the monoclonal antibody. Thus the present invention was achieved.

More specifically, the present invention is as follows:
(1) A monoclonal antibody which recognizes native SLC6A6.
(2) A monoclonal antibody which recognizes a polypeptide of an extracellular domain of SLC6A6.
(3) The monoclonal antibody according to item (2), wherein the polypeptide of the extracellular domain is represented by at least one selected from:
   (a) a polypeptide consisting of the amino acid sequence of SEQ ID NO: 8;
   (b) a polypeptide, which consists of the amino acid sequence of SEQ ID NO: 8 having substitution, deletion and/or insertion of one or several amino acids, and which functions as an extracellular domain of SLC6A6; and
   (c) a polypeptide, which consists of an amino acid sequence having at least 70% identity to the amino acid sequence of SEQ ID NO: 8, and which functions as an extracellular domain of SLC6A6.
(4) A monoclonal antibody against SLC6A6 produced by a hybridoma, wherein the accession number thereof is FERM BP-11413 or FERM BP-11414.
(5) A monoclonal antibody against SLC6A6 which binds to an epitope recognized by the monoclonal antibody according to item (4).
(6) A cell line which produces the monoclonal antibody according to any one of items (1) to (4).
(7) A cell line which produces an antibody against SLC6A6, wherein the accession number thereof is FERM BP-11413 or FERM BP-11414.
(8) A reagent for detecting SLC6A6, which comprises the monoclonal antibody according to any one of items (1) to (5).
(9) A kit for detecting or diagnosing a cancer, which comprises the monoclonal antibody according to any one of items (1) to (5).
(10) A method for detecting a cancer using the monoclonal antibody according to any one of items (1) to (5), the reagent according to item (8) or the kit according to item (9).
(11) The method according to item (10), which comprises the step of contacting a biological sample with the antibody or an antibody in the reagent or kit.
(12) The method according to item (11), wherein the biological sample is feces.
(13) The method according to item (10), wherein the cancer is a colorectal cancer.

### ADVANTAGEOUS EFFECT OF THE INVENTION

By using the monoclonal antibody of the present invention, cancer cells which significantly highly express SLC6A6 can be detected at a high sensitivity by various means. In particular, in the diagnosis of colorectal cancer, living cancer cells included in a sample from a living body such as feces can be detected simply and efficiently utilizing expression of SLC6A6 as an index. Moreover, since such an antibody can be continuously produced, it enables spread of a test kit for early detection of colorectal cancer. Furthermore, by utilizing the kit, it is possible to obtain information useful for high-precision screening of a cancer patient in a medical examination or the like, early detection of cancer, identification of the extent of cancer, monitoring of therapeutic effect at the time of therapy, prevision of metastasis/recurrence, etc., and therefore, the mortality caused by cancers can be reduced.

### BRIEF DESCRIPTION OF THE DRAWINGS

FIG. 1 shows photographs of tissue stained by in situ hybridization using a nucleotide sequence corresponding to an extracellular domain of SLC6A6 protein as a probe.
FIG. 2 shows results of analysis of expression of SLC6A6 protein by Western blotting.
FIG. 3 shows results of analysis of the titer in antisera of mice in which SLC6A6 expressing cells were transplanted by ELISA.
FIG. 4 shows results of analysis of the titer of 4B9b antibody and 5H12d antibody by ELISA.
FIG. 5 shows results of analysis of the reactivity of antibodies to cancer cell extract.
   A: results of analysis of the reactivity of 5H12d antibody to cancer cell extract
   B: results of analysis of the reactivity of 4B9b antibody to cancer cell extract
   C: results of analysis of the reactivity of polyclonal antibody to cancer cell extract
FIG. 6 shows photographs of 3 types of cancer cells stained with 4B9b antibody and 5H12d antibody.
FIG. 7 shows photographs of colorectal cancer tissue subjected to immunostaining using 4B9b antibody and 5H12d antibody.

### BEST MODE FOR CARRYING OUT THE INVENTION

Hereinafter, the present invention will be described in detail. Note that the present invention is not limited to embodiments described below, and suitable modifications thereof can be carried out without departing from the scope of the present invention.

The present invention is a monoclonal antibody which recognizes a molecule called SLC6A6 (solute carrier family 6 (neurotransmitter transpoter, taurine), member 6) or an extracellular domain thereof, and this antibody can be used, in particular, to detect colorectal cancer.

For obtaining the monoclonal antibody of the present invention, a full-length protein of human SLC6A6 or a partial protein comprising an extracellular domain thereof, amino acid residues 143-216 (SEQ ID NO: 8) (encoded by the nucleotide sequence represented by SEQ ID NO: 7), having an inherent three-dimensional structure (hereinafter sometimes collectively referred to as "protein"), is used as an immunogen. In order to obtain a form having an inherent three-dimensional structure, preferably, a cell in which the full-length protein is expressed on the cell membrane is prepared.

In general, in order to prepare an antibody, an animal is immunized by administration of a mixture of an antigen, an adjuvant, etc., whereas in the present invention, an animal is immunized by transplantation of metastatic cancer cells in order to increase immune efficiency. That is, metastatic cancer cells which express human SLC6A6 as a target antigen are transplanted and engrafted in a non-human experimental animal, and the experimental animal is immunized with the target antigen. Metastatic cancer cells are cancer cells which develop distant metastasis when transplanted in an experimental animal, and examples thereof include cancer cells having metastatic property established from bone, lung, lymph node, skin, liver, pleura, brain, breast, mammary gland, bladder, large bowel or colon. Further, by directly using metastatic cancer cells for immunization, it is possible to perform immunization with an antigen with a functional three-dimensional structure and a posttranslational modification being retained. Therefore, it is possible to induce antibodies against proteins which had difficulty in obtaining. In the present invention, cancer cells play a role in expressing an antigen, and it is possible to obtain an antibody against an antigen having an inherent three-dimensional structure (meaning a structure possessed under a living body environment). Therefore, treatments such as three-dimensional restructuring of isolated protein are not required, and it is possible to induce an antibody having sufficient neutralizing potency against an antigen.

Regarding cells, it is preferred to use cells having metastatic property which develop distant metastasis when transplanted in an experimental animal, and for example, MCF7-14 derived from MCF7 established from human breast cancer (International Publication WO2008/093886 pamphlet, Accession Number: FERM BP-10944) is used. In addition to cells having metastatic property inherent therein, cells prepared to have an acquired or artificial metastatic property using means conceived by those skilled in the art can also be used.

Expression of an antigen in cells can be performed by well-known methods in the art. For example, an expression vector comprising a polynucleotide encoding an antigen protein is constructed and the obtained expression vector is introduced into a cell, thereby allowing the target antigen protein to be expressed in the cell.

The immunogen as described above is administered to an experimental animal such as mouse and rat. Preferably, an immunodeficient mouse of the BALB/c line such as BALB/c-nu/nu or the C57BL/6 or ICR line is used.

A method of administration of the immunogen is carried out by a means, by which the immunogen is retained in the body of the experimental animal for a long period of time and immune response is caused. For example, when using protein-expressed cells, the cells are transplanted into the experimental animal. Regarding a site into which cells are transplanted, any organ or tissue in which transplanted cells are easily engrafted may be selected. When using MCF7-14, the mammary gland which belongs to the primary lesion of MCF7 from which the cell is derived, sites such as lymph node, liver, lung, bone and brain in which metastasis of breast cancer frequently occurs, tissue adjacent to the lymph node, adipose tissue and the like are preferred. In this case, a scaffold such as Matrigel (Becton, Dickinson and Company) is preferably utilized in order to enable transplanted cells to be easily engrafted.

After the administration of immunogen, the experimental animal is raised, and progression of immunization is confirmed utilizing increase of the antibody titer in the blood, enlargement of the spleen and the like as indexes. As a method for analyzing an antibody in the blood, ELISA (Enzyme-Linked ImmunoSorbent Assay) is preferably utilized.

After immunization, a monoclonal antibody is obtained according to a well-known method in the art. For example, antibody-producing cells are collected from the spleen, the lymph node or the like of the immunized experimental animal, and the cells are fused with myeloma cells to produce hybridoma cells. Clones producing an antibody having desired specificity and affinity are selected and cultured, and the antibody in supernatant is purified. In addition to this, it is also possible to intraperitoneally administer hybridoma cells to a mouse and to use ascites fluid of the mouse as a starting material to purify an antibody.

It is also possible to obtain an antibody according to a method in which hybridoma cells are not prepared. For example, antibody-producing cells collected from the spleen, the lymph node or the like are subjected to, for example, introduction of a gene (telomerase, SV40 large T antigen, E6 or E7 of HPV, E1A of adenovirus, hTERT, bmi-1, c-myc) or infection with a virus (Epstein-Barr virus (EBV), human papillomavirus (HPV), SV40) to immortalize the antibody-producing cells, and in the same manner as described above, clones are selected and then an antibody is purified. Alternatively, an antibody gene such as H chain and L chain which recognizes protein is cloned from the spleen or the lymph node of an immunized animal, the gene is subjected to gene recombination to enable the gene to encode a target antibody-like molecule, expression is caused in a host such as an animal cell, virus, yeast and bacterium, and selection is carried out, thereby preparing an antibody and an antibody-like molecule which bind to an extracellular domain of SLC6A6. The present invention also provides a cell line which produces the antibody of the present invention.

Meanwhile, it is also possible to extract an antibody gene from antibody-producing cells collected from the spleen, the lymph node or the like of the immunized experimental animal or prepared hybridoma cells to prepare an antibody-like molecule specific to an extracellular domain of SLC6A6. Specifically, in the same manner as described above, an antibody gene is isolated, the gene is suitably subjected to gene recombination using a publicly-known technique, and it is introduced into another cell to cause expression.

In the present invention, an antibody fragment that is a part of a monoclonal antibody, a smaller antibody, a recombinant antibody, and an antibody-like molecule such as a modified antibody can be prepared by various genetic engineering and protein engineering techniques. Specific examples thereof include H chain, L chain, Fv, Fab, Fab', F(ab')2, scFv, sdFv, sc(Fv)2, (scFv)2, DiAbody, chimeric antibody, humanized antibody, human antibody, single-chain antibody, multispecific antibody such as bispecific antibody, and labeled antibody. In each case, any molecule having the ability to bind to an extracellular domain of SLC6A6 is included in the monoclonal antibody of the present invention.

The monoclonal antibody of the present invention can recognize native SLC6A6. The word "native" means that the protein is in the state of having a three-dimensional structure possessed under a living body environment.

Further, the monoclonal antibody of the present invention can recognize an extracellular domain of SLC6A6. In particular, as the extracellular domain, the region of amino acid residues 143-216 of SLC6A6 (SEQ ID NO: 8) can be recognized by the monoclonal antibody.

The monoclonal antibody of the present invention may be a monoclonal antibody which recognizes: a mutant-type polypeptide in which one or several (for example, from 2 to 20, preferably from 2 to 10, and more preferably 2, 3, 4 or 5) amino acids are substituted, deleted or inserted in the amino acid sequence of SEQ ID NO: 8; and a mutant-type polypeptide having at least 70%, and preferably at least 80%, at least 90%, at least 95%, or at least 98% or at least 99% identity to the amino acid sequence of SEQ ID NO: 8, as long as the monoclonal antibody is within the range in which the activity to bind to a polypeptide having the amino acid sequence of SEQ ID NO: 8 is retained, that is, a target polypeptide to be bound has the function as the extracellular domain of SLC6A6. Further, the monoclonal antibody of the present invention may be a monoclonal antibody that recognizes a polypeptide having the function as the extracellular domain of SLC6A6, which is: a polypeptide encoded by a polynucleotide consisting of the nucleotide sequence of SEQ ID NO: 7; a polypeptide encoded by a polynucleotide comprising the nucleotide sequence of SEQ ID NO: 7; a mutant-type polypeptide encoded by a polynucleotide having at least 70%, and preferably at least 80%, at least 90%, at least 95%, at least 98% or at least 99% identity to the nucleotide sequence of SEQ ID NO: 7; or a mutant-type polypeptide encoded by a polynucleotide comprising a nucleotide sequence which hybridizes to the nucleotide sequence of SEQ ID NO: 7 under highly stringent conditions. In this regard, hybridization can be carried out according to a publicly-known method (for example, Molecular Cloning 2nd Ed (Cold Spring Harbor Lab. Press, 1989)). Highly stringent conditions mean conditions under which a so-called specific hybrid is formed and no non-specific hybrid is formed, and for example, conditions in which the sodium concentration is from 10 mM to 300 mM, and preferably from 20 mM to 100 mM and the temperature is from 25°C to 70°C, and preferably from 42°C to 55°C.

It is inferred that the extracellular domain of SLC6A6 plays a role in binding to taurine and transport of taurine into the inside of a cell. It is considered that whether or not a mutant-type polypeptide has the function as the extracellular domain of SLC6A6 can be confirmed by performing forced expression of the mutant-type polypeptide in animal cells or the like and analyzing uptake of taurine by the activation method (the method described in J. Membr. Biol Vol. 76, 1-15, 1983).

Alternatively, since the monoclonal antibody of the present invention binds to SLC6A6, when the monoclonal antibody of the present invention can bind to a mutant-type polypeptide, it indicates that the activity of the antibody to bind to a polypeptide having the amino acid sequence represented by SEQ ID NO: 8 is retained, that is, the mutant-type polypeptide is included in the polypeptide having the function as the extracellular domain of SLC6A6.

Binding of the monoclonal antibody of the present invention to a polypeptide such as a mutant-type polypeptide can be confirmed by ELISA, immunoprecipitation, western blotting or the like.

Further, the extracellular domain of SLC6A6 is a site of the cell surface of marker protein whose expression is increased in cancer cells. Whether or not a polypeptide such as a mutant-type polypeptide has the function as the extracellular domain of SLC6A6 can be confirmed by comparison of expression of the polypeptide in normal cells and cancer cells by means of immunostaining, ELISA, immunoprecipitation, western blotting or the like. The matter that the monoclonal antibody of the present invention recognizes native SLC6A6 can also be confirmed by utilizing the above-described method, but it is not limited to the above-described method as long as binding of the monoclonal antibody of the present invention to SLC6A6 having a three-dimensional structure possessed in the living body can be detected.

The monoclonal antibody of the present invention has higher affinity to SLC6A6 compared to conventional antibodies.

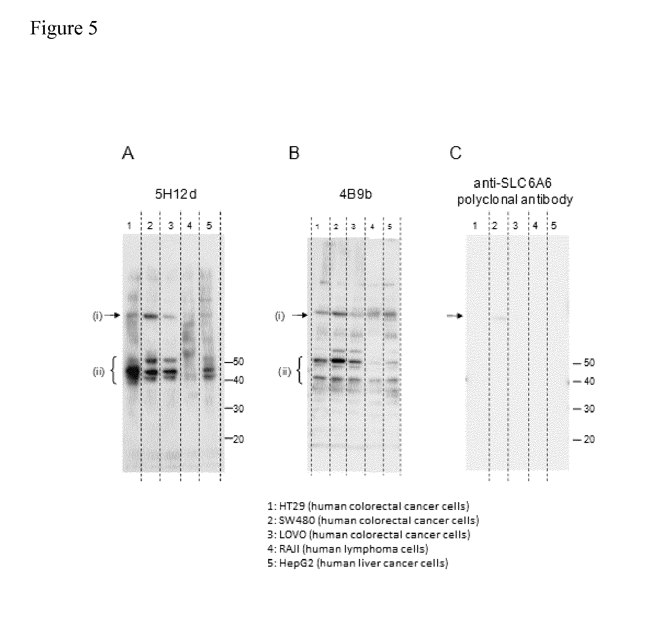

Conventional antibodies do not recognize an extracellular domain, and therefore it is difficult to utilize them because, for example, they cannot bind to living cells, a detection signal thereof is very weak, and it is impossible to stably produce homogenous antibodies. Whereas unlike such conventional antibodies, the monoclonal antibody of the present invention recognizes an extracellular domain, and in addition, has higher affinity compared to conventional antibodies. Therefore, the antibody of the present invention can detect SLC6A6 present on the surface of cancer cell at a higher sensitivity and can catch a tiny amount of cancer cells contained in a sample. As a result, a cancer can be early found (see Figure 5).

The monoclonal antibody of the present invention also recognizes a protein encoded by an mRNA variant of slc6a6 (see Figure 5). The antibody of the present invention can bind not only to the full-length SLC6A6, but also to a mutant in which a portion thereof is deficient, and therefore, the antibody can detect a wider range of cancer cells expressing SLC6A6 compared to the conventional antibodies.

As a cell line (hybridoma) which produces the monoclonal antibody of the present invention, "4B9b" and "5H12d" are exemplified. Both were deposited under the Budapest Treaty to International Patent Organism Depositary, National Institute of Advanced Industrial Science and Technology (Chuo 6, Higashi 1-1-1, Tsukuba-shi, Ibaraki 305-8566) on July 21, 2010 by Bio Matrix Research, Inc. (105, Higashifukai, Nagareyama-shi Chiba, 270-0101). The accession number of "4B9b" is "FERM BP-11413" and the accession number of "5H12d" is "FERM BP-11414" (date of the original deposit: July 21, 2010). The present invention provides the hybridomas and antibodies produced by them. By culturing the hybridomas, homogenous monoclonal antibodies can be produced.

The monoclonal antibody of the present invention having the above-described characteristics can be efficiently obtained by using the immunization method in which cells expressing a protein are engrafted as described above, which is different from usual methods for producing a monoclonal antibody. It is difficult to prepare an antibody against a membrane protein such as SLC6A6, particularly against a multi-transmembrane protein using a production method generally employed by those skilled in the art mainly for the following reasons:
(1) When a surfactant is used for preparing a membrane protein as an antigen, the membrane protein loses its three-dimensional structure, meanwhile when no surfactant is used for this purpose, the membrane proteins aggregate through their hydrophobic regions;
(2) No immune response is induced because the expression level on the cell surface is low or an extracellular domain is small.

Original ideas were applied to the antibody production method (particularly the immunization method) by the present inventors, and as a result, the monoclonal antibody of the present invention has excellent characteristics when compared to conventional antibodies.

The monoclonal antibody of the present invention has characteristics described below.

The antibody of the present invention can recognize native SLC6A6 because the antibody is prepared based on the three-dimensional structure originally possessed by SLC6A6. Therefore, the antibody of the present invention has a significantly higher detection sensitivity compared to the conventional antibody (HPA015028) that recognizes the same epitope, and can sufficiently detect a slight amount of SLC6A6, which was difficult for the conventional antibody to detect. Further, the conventional monoclonal antibody (sc-166640) recognizes a region in the membrane and cell of SLC6A6, whereas the antibody of the present invention recognizes an extracellular site, and therefore, the antibody of the present invention can bind to living cells and can detect cancer cells in a sample after a simple pretreatment. Moreover, the antibody of the present invention can detect SLC6A6 or cancer cells qualitatively and/or quantitatively in various detection methods such as immunostaining, western blotting and ELISA, and can clearly distinguish a site of expression in cells, tissue or the like. In addition, the conventional antibody is a polyclonal antibody, and it was difficult to continuously produce homogenous antibodies, but the antibody of the present invention is a monoclonal antibody, and therefore it is possible to mass-produce antibodies with good reproducibility. By virtue of the above-described characteristics, the antibody of the present invention is useful for detection of cancer, and early detection of cancer can be realized by utilizing the antibody in the form of a test kit or the like for diagnosis.

Further, the present invention is not limited to the very monoclonal antibody against SLC6A6 produced by the hybridoma whose accession number is FERM BP-11413 or FERM BP-11414, and any product which binds to an epitope recognized by the monoclonal antibody produced by the hybridoma is included in the monoclonal antibody against SLC6A6 of the present invention. The "epitope" as used herein refers to an epitope recognized by the monoclonal antibody produced by the above-described hybridoma (it may be amino acid residues 143-216 in the amino acid sequence of SLC6A6 or a portion of the region).

By using the monoclonal antibody of the present invention, unlike the conventional antibodies, it is possible to detect expression of SLC6A6 in living cells or floating cells in a suspension without changing the form and characteristics of cells in a sample.

When using an antibody which recognizes a transmembrane region or intracellular region at the time of detection of protein, in general, a sample such as a tissue slice and cells is fixed using formalin, methanol or the like and the cell membrane is permeabilized using a surfactant. However, by such a treatment, the protein is denatured and the inherent three-dimensional structure thereof collapses, and therefore, the detection sensitivity is reduced. Moreover, it is required to improve the detection sensitivity by means of activation of antigen and rigorous control of fixing conditions, and conditions for staining the antibody are limited. Meanwhile the antibody of the present invention can detect SLC6A6 even in a tissue slice fixed in a manner similar to that in the case of the conventional antibodies or a sample in which the cell membrane is destroyed, and in addition, can detect SLC6A6 present on the cell membrane without such a treatment at a high sensitivity. Therefore, the range of detection means to which the antibody can be applied is wide.

The monoclonal antibody of the present invention can be used for detection of cancer cells which express SLC6A6. The present invention provides a method for detecting a cancer using the monoclonal antibody of the present invention, a reagent for detection of SLC6A6 comprising the monoclonal antibody of the present invention, and a kit for detection or diagnosis of a cancer comprising the monoclonal antibody of the present invention. A target cancer may be any cancer when SLC6A6 is significantly highly expressed in tumor cells compared to normal tissue, and colorectal cancer, stomach cancer, bladder cancer, kidney cancer, uterus cancer and breast cancer are suitable.

A sample as a test target in the detection method of the present invention is not particularly limited as long as it is a biological sample closely associated with a target cancer in a test. For example, when testing colorectal cancer or stomach cancer, cancer tissue, body fluid, excrement and the like can be utilized, and feces is particularly preferred. In the case of bladder cancer or kidney cancer, cancer tissue, urine, etc. can be utilized, and in the case of uterus cancer and breast cancer, a sanitary product, breast milk, etc. can be utilized. Any biomaterial can be adapted by a suitable pretreatment.

When using feces as a sample, it is preferably prepared according to the method described in Patent Document 1. According to the method, cancer cells can be efficiently collected from feces. Specifically, feces and a buffer are put into a stomacher bag to prepare a suspension of feces. The suspension is filtered using a multistage filter apparatus, and with the pore of the final filter being set to 10 µm or less, cells are captured on the final filter. Among them, cells are collected using Ber-EP4 antibody-bound magnetic beads (Dynabeads Epithelial Enrich, Dynal). Other than this, methods for efficiently collecting cells usually used by those skilled in the art, including the Percoll centrifugation method (Japanese National-phase PCT Laid-Open Patent Publication No. H11-511982), may be utilized.

Regarding various cancers including colorectal cancer, no tumor marker which realizes early detection of such cancers using body fluid has been established. General markers sometimes show false negative even in the case of advanced cancer. Meanwhile, when using the antibody of the present invention, it is possible to detect and/or measure a slight amount of cells which express SLC6A6 as a marker included in the body fluid or excrement prior to cancer progression.

The detection method of the present invention uses the monoclonal antibody of the present invention, and comprises the step of contacting a biological sample with the monoclonal antibody of the present invention (an antibody produced from the hybridoma, or an antibody included in the reagent or kit of the present invention). Further, the term "contact" means that the antibody of the present invention and a biological sample as a test target (suitably pretreated according to need) are kept under conditions in which they react with each other, and mixing the antibody and the biological sample in a well for reaction, adding the antibody to tissue or cells as a test sample, adding one of the test sample and the antibody to a membrane to which the other is adsorbed, and adding one of the test sample and the antibody to a carrier such as a resin to which the other is fixed are all included in the "contact".

Further, as a technique for detection, any technique may be used as long as it measures or detect SLC6A6 utilizing specificity of antigen-antibody reaction. For example, enzyme immunoassay (EIA), fluorescence immunoassay (FIA), radioimmunoassay (RIA), chemiluminescent immunoassay (CIA), turbidimetric immunoassay, nephelometric immunoassay, latex agglutination, latex turbidimetry, hemagglutination reaction, particle agglutination, Western blotting, immunostaining, immunoprecipitation, the immunochromato method, ELISA and the like can be utilized.

A device to be used for such detection is not particularly limited, and for example, a microwell plate, an array, a chip, a flow cytometer, a surface plasmon resonance apparatus, an immunochromatography strip and the like can be utilized. When using such a device, a signal such as the amount of color development, the amount of fluorescence, the amount of luminescence and the like is used as an index of detection, and it can be correlated with existence of cancer, cancer progression, etc.

When carrying out the detection method of the present invention using enzyme immunoassay or fluorescence immunoassay, for example, the above-described sample, such as cells including cancer cells collected from feces and a section prepared from tissue collected from a patient, is suitably pretreated, and the pretreated sample is immobilized to a solid phase such as a microwell plate and a slide glass to perform immunological reaction. Alternatively, it is also possible to react a cell suspension with the antibody in a tube. Further, it is also possible to utilize a method in which the monoclonal antibody of the present invention is immobilized to beads or a microwell plate and reacted with cells.

In this case, the monoclonal antibody of the present invention may be labeled with an enzyme or fluorescent substance to directly detect the reaction as a fluorescence signal. Alternatively, a labeled secondary antibody that binds to the monoclonal antibody of the present invention may be used to indirectly detect the signal. As a labeling substance, a substance usually used by those skilled in the art such as peroxidase (POD), alkaline phosphatase, β-galactosidase and biotin-avidin complex may be utilized. Further, a method for detection may be any method, for example, the competition method, the sandwich method, the direct adsorption method or the like. Further, the reaction strength and the ratio of cells bound to the antibody in all the cells in the sample reacted are measured, and results thereof are compared to reference values or indexes set in advance, thereby determining the possibility of being affected with a cancer.

An antibody to be used for detection may be a usual monoclonal antibody, and may also be in the form of an antibody fragment, a smaller antibody, a recombinant antibody and an antibody-like molecule such as a modified antibody, for example, Fab obtained by the papain treatment, F(ab')2 or F(ab') obtained by the pepsin treatment or the like, as long as it is a molecule having the ability to bind to an extracellular domain of SLC6A6.

According to the detection method of the present invention, a sample from a cancer patient can be distinguished from a sample from a healthy subject, and therefore, the possibility of being affected with a cancer can be determined in a specific sample.

Further, the present invention provides a kit for detecting or diagnosing a cancer comprising the antibody of the present invention for carrying out cancer detection using the detection method of the present invention.

By detecting SLC6A6 included in a sample collected from a subject using the kit of the present invention, being affected with a cancer can be diagnosed. A target cancer to be diagnosed may be any cancer when SLC6A6 is significantly highly expressed in tumor cells compared to normal tissue. Examples thereof include colorectal cancer, stomach cancer, bladder cancer, kidney cancer, uterus cancer and breast cancer, and colorectal cancer is particularly suitable.

Materials constituting the kit of the present invention are not particularly limited as long as the kit comprises the monoclonal antibody of the present invention. For example, in the case of detection using enzyme immunoassay, the kit may comprise an enzyme and a substrate, various buffers, a reaction solution, a standard substance, etc. in addition to the monoclonal antibody of the present invention. The reagent may be provided in the state of being fixed, in an aqueous solution, in the state of being lyophilized or the like, and prepared into a suitable state before use.

By using the kit of the present invention, it is possible to obtain information useful for screening of a cancer patient in a medical examination or the like, identification of the extent of cancer, monitoring of therapeutic effect at the time of therapy, prevision of metastasis/recurrence, etc.

### EXAMPLES

### Example 1: Analysis of expression of SLC6A6 gene

For the purpose of identifying a new cancer cell-specific membrane protein and preparing an antibody to be used for diagnosis and the like, a new membrane protein marker was identified. Genes which were expressed in 5 types of cultured cell lines of colorectal cancer (HT29, HCT116, DLD1, LOVO, SW480) and not expressed in 2 types of normal cells (normal cells removed from large bowel derived from 2 healthy subjects subjected to colonoscopy) were identified by DNA microarray.

Among the obtained 180 candidate genes, 25 genes, in which the difference between the expression level in caner sites and in healthy sites of 5 cases was twice or more, were selected by the quantitative PCR method. Among the genes, a plurality of genes in which cancer site-specific transcription was shown were identified by in situ hybridization method. Among them, genes which are not expressed in any of the 39 types of normal tissues published on the database of Laboratory for Systems Biology and Medicine, the University of Tokyo (http://www.lsbm.org/) were examined, and solute carrier family 6 (neurotransmitter transporter, taurine, SLC6A6) gene was identified.

In order to confirm expression of SLC6A6 gene in a cancer site and a healthy site, a probe against the sequence positioned at 5461-5878 (418 bp) of mRNA (NM_003043) was prepared and tissues were analyzed according to the in situ hybridization method.

A paraffin section of colorectal cancer tissue (Genostaff Co. Ltd.) was subjected to the xylene treatment, and then hydrated with ethanol and PBS in this order, and it was fixed with paraformaldehyde for 15 minutes. It was treated with 7 µg/ml Protreinase K (Roche) for 30 minutes and fixed again with 4% paraformaldehyde solution. It was acetylated with 0.25% acetic anhydride and 0.1 M Tris-HCl (pH 8.0) for 10 minutes and dehydrated with ethanol. It was reacted with a hybridization reaction solution (Genostaff Co. Ltd.) containing 300 ng/ml probe (Genostaff Co. Ltd.) at 60°C for 16 hours, and then washed with 5x wash solution (Genostaff Co. Ltd.) at 60°C for 20 minutes and with 50% formamide/2x wash solution at 60°C for 20 minutes. Then it was treated with RNaseA at 37°C for 30 minutes.

It was washed with 2x wash solution and TBS-T and then reacted serially with 0.5% blocking reaction solution (Roche) and 20% heat-treated sheep serum (Sigma). It was reacted with AP-labeled anti-DIG antibody (Roche) for 2 hours and washed with PBS. After that, it was subjected to color development in a NBT/BCIP solution (Roche). It was counterstained with a Kernechtrot solution (Mutoh), dehydrated, and then mounted with Malinol (Mutoh). After that, it was observed with a microscope.

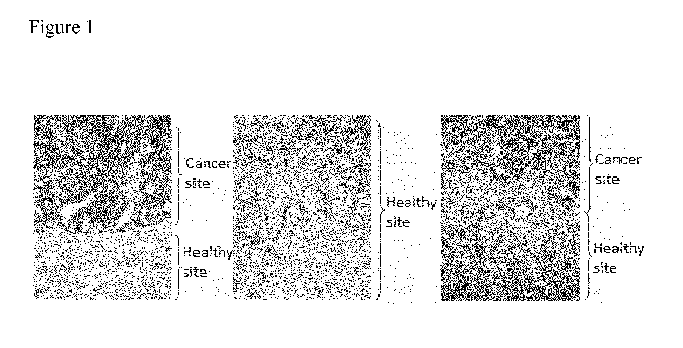

Results are shown in Figure 1. As shown in Figure 1, the cancer site of colorectal cancer was specifically stained compared to the healthy site.

### Example 2: Preparation of monoclonal antibody and detection of cancer cells

### (1) Cells

As MCF7-14, a cell line obtained by subculture of FERM BP-10944 was used. HT-29, SW480 and LOVO were obtained from National Cancer Center Hospital East.

MCF7-14 was cultured and subcultured in a RPMI1640 medium (Sigma) containing 10% (v/v) serum (Hyclone), and the other cells were cultured and subcultured in a DMEM medium (Sigma). Each of them was cultured and subcultured under 5% CO₂ at 37°C for 48 to 72 hours at not more than 80% confluence.

### (2) Cloning of SLC6A6 gene

MCF7-14 was cultured and the total RNA was extracted using RNeasy Mini kit manufactured by Qiagen. 2 µg of the extracted total RNA was subjected to a reverse transcription (RT) reaction at 50°C for 1 hour using SuperScript III reverse transcriptase (Invitrogen) to synthesize a cDNA, and the reaction was terminated by heating at 85°C for 5 minutes. Using the obtained cDNA as a template, a PCR reaction was performed using the following primers:

### Primer Sequences

Forward: AAAGGATCCATGGCCACCAAGGAGAAGCTGC (SEQ ID NO: 9)
Reverse: AAATCTAGACATCATGGTCTCCACAATGATGTG (SEQ ID NO: 10)

Regarding the PCR reaction, preincubation was performed at 95°C for 10 minutes, and then a cycle of denaturation at 95°C for 15 seconds and annealing/elongation at 60°C for 1 minute was performed 40 times, thereby amplifying a gene fragment.

The obtained amplified fragment was incorporated into a pEF6 vector (Invitrogen) using restriction enzymes (BamHI and XbaI) positioned on the primers. The amplified fragment was confirmed by DNA sequencing, and it was confirmed that the same gene sequence as that of the database was incorporated and that the c-myc tag sequence was added to the C terminus.

### (3) Preparation of SLC6A6-overexpressed line

The plasmid prepared in (2) above was introduced into MCF7-14 cells using FUGENE6 (Roche Applied Science). The operation was carried out based on the material attached to the kit.

The cells were cultured in the medium described in Example 2 (1) above with 10 µg/mL of Blasticidin S hydrochloride being added, the medium was replaced every 3-5 days, and cells having drug resistance were selected. In order to select SLC6A6-overexpressed cells from the obtained resistant strains, each of the cultured MCF7-14 cells and the gene-introduced cells were seeded in a 96-well plate at 80% confluence and cultured under 5% CO₂ at 37°C for 16 hours.

The culture supernatant was removed, and then 100 µL of 10% (v/v) neutral buffered formalin solution (WAKO) was added, and a reaction was performed at room temperature for 10 minutes. After removal of the formalin solution, it was washed with PBS (-) 3 times and air-dried, thereby preparing a plate with each of the cells being immobilized thereto.

An anti-c-myc antibody (Santa Cruz, clone 9E10) was diluted to 1 µg/mL with TBS-T (25 mM Tris, 150 mM NaCl, 0.05% (v/v) Tween20, pH 7.4), and as a primary antibody, it was added to the immobilized plate in an amount of 100 µL per well, and it was reacted at room temperature for 1 hour. Each well was washed with 200 µL of TBS-T 3 times.

As a secondary antibody, anti-mouse IgG polyclonal antibody-HRP label (BETHYL) was diluted 5,000-fold with TBS-T. This antibody dilution was added in a volume of 100 µL per well and reacted at room temperature for 30 minutes. Each well was washed with 200 µL of TBS-T 3 times.

Orthophenylenediamine (Sigma) was diluted with 50 mM carbonate-citrate buffer (pH 5.0) to give a final concentration of 0.5 mg/mL, and mixed with 1/10,000 volume of 35% (w/w) aqueous hydrogen peroxide (WAKO). This mixture was added as a substrate solution in a volume of 100 µL per well and reacted at room temperature for 10 minutes. 25 µL of 3N sulfuric acid (WAKO) was added thereto to terminate the reaction. The absorbance at 492 nm was measured with a plate reader (SpectraMaxPure384, Molecular Devices) to examine signals, and 3 types of strains showing a signal higher than MCF7-14 were selected.

### (4) Western blot

The 3 types of cells obtained in (3) above were cultured on a 10 cm petri dish to give 90% confluence and washed with PBS (-) (0.01 M sodium-phosphate buffer, 0.138 M NaCl, 0.0027 M KCl, pH 7.4) twice. To the cells, 200 µL of 2x RIPA Buffer (0.1 M Tris, 0.3 M EDTA, 1% (v/v) Triton X-100, 2% (w/v) Sodium Deoxycholate, 0.2% (w/v) sodium dodecyl sulfate) was added, and it was left on ice for 1 minute. After that, cell solution was collected using a scraper. The cells were broken for 30 seconds using an ultrasonic breaking machine (Branson) to obtain an extract. With respect to each of the cells, an extract was prepared, and the protein concentration was measured according to the Bradford method. After that, it was prepared to give the same protein content and subjected to SDS-PAGE. After electrophoresis, using Trans-Blot SD Cell (Bio-Rad), the protein was transferred to a PVDF membrane (PIERCE) according to the protocol recommended by the manufacturer. Using 5% (w/v) skim milk dissolved in TBS-T, blocking was performed at room temperature for 30 minutes, and it was washed with TBS-T twice. A c-myc antibody was diluted to 1 µg/mL with TBS-T and reacted with the membrane at room temperature for 1 hour. It was washed with TBS-T 3 times, and then as a secondary antibody, anti-mouse IgG polyclonal antibody-HRP label (BETHYL) was diluted 10,000-fold with TBS-T. It was reacted with the membrane at room temperature for 30 minutes, and then washed with TBS-T 3 times. The membrane was immersed in Immobilon (Millipore) and then wrapped, and a signal was detected using LAS-3000 (Fujifilm Corporation).

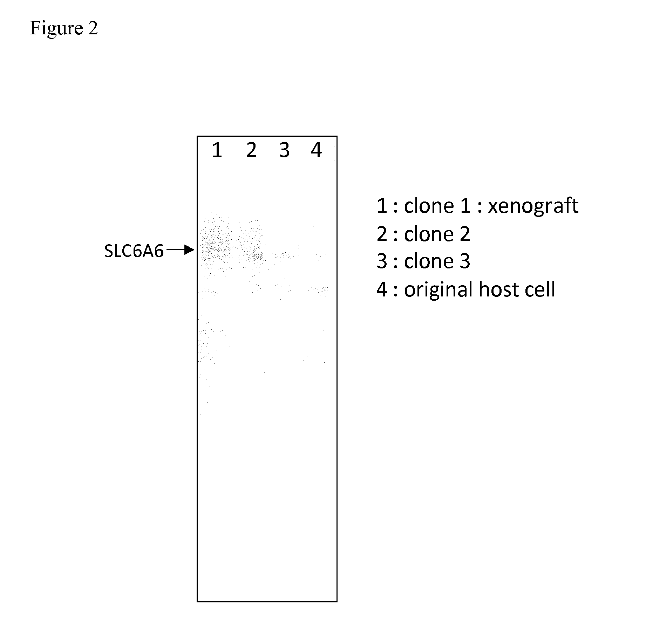

Results are shown in Figure 2.

Figure 2 shows results of analysis of protein expression with respect to SLC6A6 gene-introduced cells (1-3) and a cell without gene introduction (4). Clone 1, which showed most prominent expression, was used in the experiment described below.

### (5) Cell transplantation

Cells were cultured on a 10 cm petri dish to give 90% confluence and collected using trypsin (GIBCO), and then washed with PBS (-) (0.01 M sodium-phosphate buffer, 0.138 M NaCl, 0.0027 M KCl, pH 7.4) twice. After washing, the cells were suspended in Growth Factor Reduced Matrigel (Becton Dickinson) to give a final concentration of 8.6 × 10⁷ cells/mL, and preserved on ice until transplantation.

Chloral hydrate (Sigma) was dissolved at a concentration of 3.5% (w/v) in physiological saline to prepare a 3.5% solution of chloral hydrate in physiological saline. 6 to 8-week-old nude mice (BALB/cALcl-nu/nu line (CLEA Japan, Inc.)) were anesthetized by being intraperitoneally administered with 0.2 mL of the 3.5% solution of chloral hydrate in physiological saline. Into the fourth mammary gland in each mouse, the cells suspended in the Matrigel were transplanted in an amount of 1 × 10⁶ cells per mammary gland via a 24G injection needle, such that the cells did not extend off the mammary gland. Each mouse received two transplantations, one at left and another at right fourth mammary gland in the trunk.

### (6) Expression and Purification of SLC6A6 partial protein for screening

From the full-length gene of SLC6A6 introduced into the pEF6 vector described in (3) of Example 2, the region of amino acid residues 143-216 (SEQ ID NO: 8), as an extracellular domain, was subcloned into a pET32 vector. A PCR reaction was performed using the following primers:

### Primer Sequences

Forward: ATAGGATCCGGCCTGGGCCACATATCACCTG (SEQ ID NO: 11)
Reverse: TATGAATTCGCTTTCAGAGAGCCTGGGTGGTC (SEQ ID NO: 12)

Regarding the PCR reaction, preincubation was performed at 94°C for 2 minutes, and then a cycle of denaturation at 98°C for 10 seconds, annealing at 58°C for 30 seconds and elongation at 68°C for 30 seconds was performed 30 times, thereby amplifying a gene fragment.

The obtained amplified fragment was incorporated into a pET32 vector (Novagen) using restriction enzymes (EcoRI and BamHI) positioned on the primers.

The nucleotide sequence of the amplified fragment was confirmed by DNA sequencing, and it was confirmed that the same gene sequence of the extracellular domain as that of the database was incorporated and that the His tag sequence was added to the C terminus.

BL21 (DE3) (Invitrogen) was transformed with this vector and cultured in LB medium (1% (w/v) tryptone (Sigma), 0.5% (w/v) Yeast extract (Sigma), 0.5% (w/v) NaCl (Sigma)) containing 1% (w/v) glucose. After the turbidity of the medium at 600 nm reached 0.6, 1 mM IPTG (WAKO) was added, and it was cultured for 16 hours. The cells were collected by centrifugation and then subjected to ultrasonic breaking, thereby obtaining a fraction comprising the extracellular domain of SLC6A6 as an insoluble protein.

About 10 mg of the sample was dissolved in Buffer A (1M guanidine hydrochloride (Sigma), 10 mM DTT (Sigma), 10 mM EDTA (Sigma)) and reacted at 37°C for 1 hour. The reaction solution was slowly added to 1 L of Buffer B (50 mM Tris, 150 mM NaCl, 5% glycerol, 0.4 mM oxidized glutathione (Sigma), pH 8.5), and the mixture was stirred at 4°C for 18 hours. The dissolved sample was applied to a Ni sepharose column (GE) and eluted with Buffer C (50 mM potassium phosphate buffer, 150 mM NaCl, 200 mM Imidazole, pH 8.0). It was dialyzed against Buffer C not containing Imidazole, thereby obtaining a purified partial protein of the extracellular domain of SLC6A6.

### (7) Analysis of antiserum

100 µL of the recombinant protein obtained in (6) above (10 µg/ml) or 100 µL of PBS was put into each well of a MaxiSorp 96-well plate (Nunc) and adsorbed to the plate at room temperature for 1 hour. After adsorption, the wells were washed with TBS-T (25 mM Tris, 150 mM NaCl, 0.05% (v/v) Tween20, pH 7.4), and after that, 5% skim milk dissolved in TBS-T (GIBCO) was put into them and blocking was performed at room temperature for 30 minutes. Each well was washed with 200 µL of TBS-T 3 times. After that, plasma collected from the tail vein of the mouse subjected to transplantation in (5) above was subjected to 1/2000 dilution with TBS-T, and 100 µL thereof was added to each well of an ELISA plate and reacted at room temperature for 1 hour. Each well was washed with 200 µL of TBS-T 3 times.

As a secondary antibody, anti-mouse IgG polyclonal antibody-HRP label (BETHYL) was diluted 5,000-fold with TBS-T. This antibody dilution was added in a volume of 100 µL per well and reacted at room temperature for 30 minutes. Each well was washed with 200 µL of TBS-T 3 times.

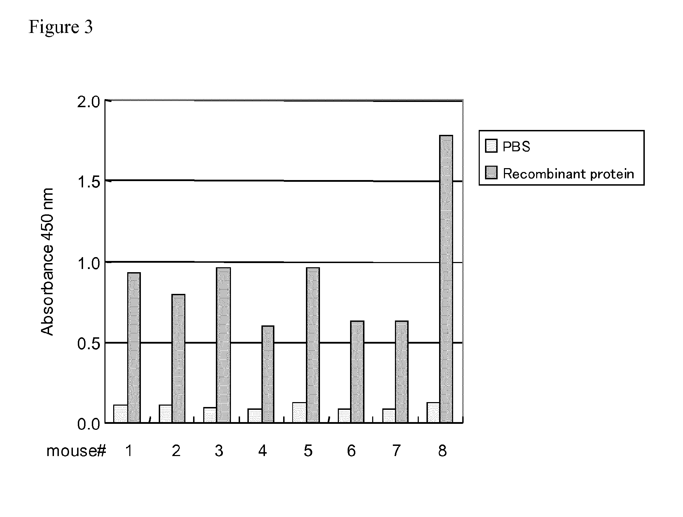

Orthophenylenediamine (Sigma) was diluted with 50 mM carbonate-citrate buffer (pH 5.0) to give a final concentration of 0.5 mg/mL, and mixed with 1/10,000 volume of 35% (w/w) aqueous hydrogen peroxide (WAKO). This mixture was added as a substrate solution in a volume of 100 µL per well and reacted at room temperature for 10 minutes. 25 µL of 3N sulfuric acid (WAKO) was added thereto to terminate the reaction. The absorbance at 492 nm was measured with a plate reader (SpectraMaxPure384, Molecular Devices) to analyze the titer of the antibody (Figure 3).

### (8) Cell fusion

Lymphocytes derived from the spleen of the mouse, regarding which the titer in antiserum was confirmed in (7) above, were fused with mouse myeloma cell line P3X63-Ag8 (ATCC Accession No. CRL-1580) according to the conventional method using 50% (v/w) polyethylene glycol 4000 (Sigma).

The fused cells were suspended in HAT medium (Invitrogen) and dispensed into twenty 96-well plates in a volume of 100 µL per well. During culture, 200 µL of HAT medium was added to each well. After culture for 11 to 16 days, the plates were observed under a microscope, indicating that 1 to 6 colonies were formed per well.

### (9) Obtaining of monoclonal antibodies

7 months after transplantation, spleen cells were collected and used to prepare hybridoma cells in the same manner as that described in (8) above. In order to select antibodies recognizing SLC6A6, clones were selected using the methods described in (3) and (7) above. As a primary antibody, culture supernatant of cells was used, and as a secondary antibody, anti-mouse IgG polyclonal antibody-HRP label was used.

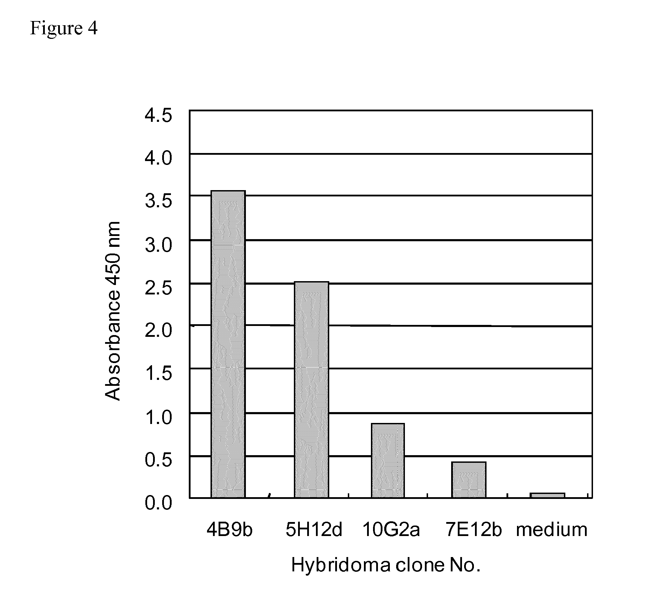

### Results are shown in Figure 4.

Figure 4 shows results obtained by analyzing the obtained clones according to the same method as that described in (7) above. In the analysis described below, antibodies produced by 2 hybridoma cells of clones 4B9b and 5H12d were used. Hereinafter, antibodies produced by the respective hybridoma cells are sometimes referred to as 4B9b antibody and 5H12d antibody.

### (10) Western blot

In order to analyze characteristics of 4B9b antibody and 5H12d antibody, detection of SLC6A6 was carried out using 3 types of colorectal cancer cells (HT29, SW480, LOVO) according to the same Western blot method as that in (4) above. As a control for comparison, a polyclonal antibody HPA015028 (Atlas) was used. Each of the antibodies was purified for use (1 µg/mL).

Results are shown in Figure 5. In the case of the full-length protein of SLC6A6, a band appears in the proximity of (i). Regarding HPA015028 (Figure 5C), SLC6A6 was successfully detected in SW480 and LOVO, but SW480 showed a weak signal and LOVO showed a very weak signal. In HT29, no signal of SLC6A6 was successfully detected. Regarding 4B9b (Figure 5B) and 5H12d (Figure 5A), SLC6A6 was successfully detected in all the types of the colorectal cancer cells, and it is understood that the detection sensitivities thereof are significantly higher than that of the polyclonal antibody HPA015028. In fact, the signal was clearly detected in HT29, though it was not seen in the case of HPA015028. In addition, unlike HPA015028, a plurality of bands of variants having a low molecular weight with a deletion in the C-terminal side (mainly in the proximity of (ii)) were observed. This clearly shows that the detection sensitivity of the polyclonal antibody HPA015028 is low, whereas the detection sensitivity of the monoclonal antibody of the present invention is high.

### (11) Immunostaining

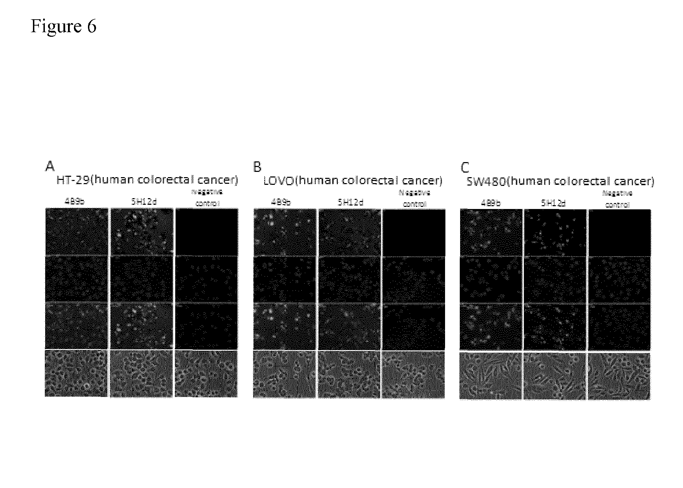

Using 4B9b antibody and 5H12d antibody, 3 types of colorectal cancer cells (HT29, SW480, LOVO) were immunostained. The cells were cultured on a glass and reacted with the antibodies according to the method described in (3) above. As primary antibodies, culture supernatants of 4B9b and 5H12d were used. As a secondary antibody, Alexa488-labeled anti-mouse IgG (Becton Dickinson) was subjected to 1/2500 dilution for use. Observation was carried out using a fluorescence microscope (Figure 6A-C). Figure 6A-C show, from top down, fluorescence signal (antibody-specific reaction), Hoechst33342 staining (nucleus), merge (superimposing), and visible light.

The results in Figure 6 show that the monoclonal antibody of the present invention recognizes SLC6A6 in immunostaining of cancer cells and therefore can be used for detection of SLC6A6 or cancer cells having SLC6A6 by immunostaining.

### (12) Histological stain

Immunostaining was carried out using 4B9b antibody and 5H12d antibody. Human colorectal cancer tissue (Biochain) was fixed using paraffin and then cut into a slice. It was subjected to the deparaffinization treatment using xylene and hydrophilized with ethanol. It was treated with 0.3% (v/v) hydrogen peroxide solution for 20 minutes and then washed with TBS-T 3 times. Then it was treated with steam under pressure (120°C) for 10 minutes to perform antigen activation. It was treated with PBS containing 3% (w/v) BSA and then reacted with 4B9b antibody and 5H12d antibody at 4°C for 16 hours. It was washed with TBS-T 3 times and then reacted with peroxidase-labeled anti-mouse secondary antibody (DAKO) at room temperature for 1 hour. It was washed with TBS-T 3 times and then subjected to color development using DAB reagent (DAKO) for 5 minutes. It was washed with distillated water and then subjected to nuclear staining using Hematoxylin (WAKO). It was washed with running water, treated with ethanol and xylene in this order, and then mounted.

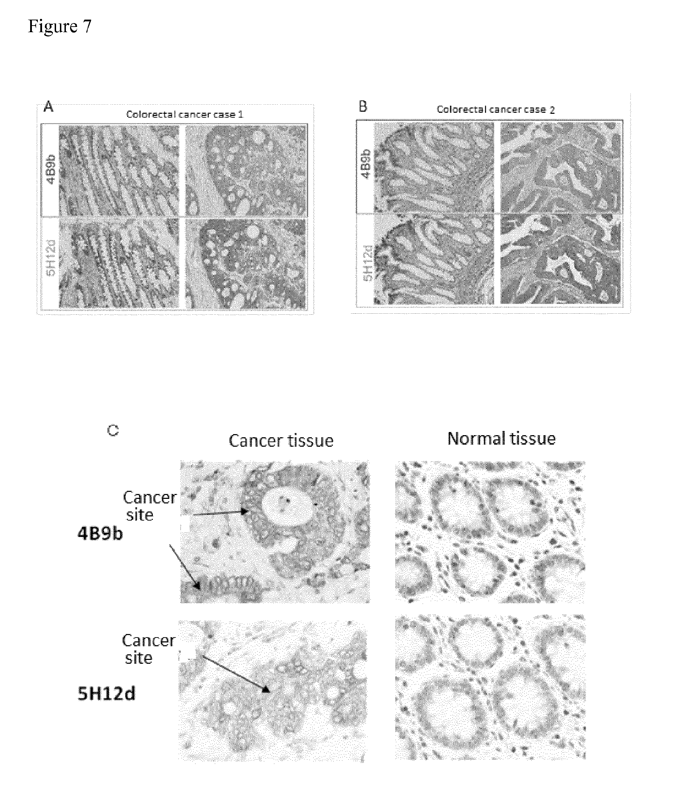

Figure 7 shows results of immunostaining with respect to 2 cases (Figure 7A-B) and results of immunostaining with respect to cancer tissue and normal tissue (Figure 7C). It is shown that the cancer sites were specifically stained.

Note that the documents, laid-open publications, patents and other patent documents cited herein are incorporated herein by reference. Further, the disclosures in the claims, specification, drawings and abstract of Japanese Patent Application No. 2010-195926 filed on September 1, 2010, to which priority is claimed by the present application, are incorporated herein by reference in their entirety.

### INDUSTRIAL APPLICABILITY

The present invention provides a monoclonal antibody which specifically binds to an extracellular domain of SLC6A6. The antibody of the present invention can be used for specific detection and measurement of cells expressing SLC6A6.

The detection method of the present invention can be utilized for diagnosis of various cancers including colorectal cancer reflected in the expression level of SLC6A6.

By detecting or quantitating SLC6A6 in a biological sample collected from a subject using the detection method and test kit of the present invention, it is possible to provide screening of a patient with suspected cancer, prediction of cancer, and judgment of conditions and progression.

### Sequence listing free text

SEQ ID NO: 9: Synthetic DNA
SEQ ID NO: 10: Synthetic DNA
SEQ ID NO: 11: Synthetic DNA
SEQ ID NO: 12: Synthetic DNA

## Claims

1. A monoclonal antibody which recognizes native SLC6A6.

2. A monoclonal antibody which recognizes a polypeptide of an extracellular domain of SLC6A6.

3. The monoclonal antibody according to claim 2, wherein the polypeptide of the extracellular domain is represented by at least one selected from:
(a) a polypeptide consisting of the amino acid sequence of SEQ ID NO: 8;
(b) a polypeptide, which consists of the amino acid sequence of SEQ ID NO: 8 having substitution, deletion and/or insertion of one or several amino acids, and which functions as an extracellular domain of SLC6A6; and
(c) a polypeptide, which consists of an amino acid sequence having at least 70% identity to the amino acid sequence of SEQ ID NO: 8, and which functions as an extracellular domain of SLC6A6.

4. A monoclonal antibody against SLC6A6 produced by a hybridoma, wherein the accession number thereof is FERM BP-11413 or FERM BP-11414.

5. A monoclonal antibody against SLC6A6 which binds to an epitope recognized by the monoclonal antibody according to claim 4.

6. A cell line which produces the monoclonal antibody according to any one of claims 1 to 4.

7. A cell line which produces an antibody against SLC6A6, wherein the accession number thereof is FERM BP-11413 or FERM BP-11414.

8. A reagent for detecting SLC6A6, which comprises the monoclonal antibody according to any one of claims 1 to 5.

9. A kit for detecting or diagnosing a cancer, which comprises the monoclonal antibody according to any one of claims 1 to 5.

10. A method for detecting a cancer using the monoclonal antibody according to any one of claims 1 to 5, the reagent according to claim 8 or the kit according to claim 9.

11. The method according to claim 10, which comprises the step of contacting a biological sample with the antibody or an antibody in the reagent or kit.

12. The method according to claim 11, wherein the biological sample is feces.

13. The method according to claim 10, wherein the cancer is a colorectal cancer.


SEQUENCE LISTING

<110> Bio Matrix Research, Inc.
National Cancer Center

<120> Antibodies specific for marker of colorectal cancer

<130> PCT11-0052

<150> JP2010-195926
<151> 2010-09-01

<160> 12

<170> PatentIn version 3.1

<210> 1
<211> 6516
<212> DNA
<213> Homo sapiens

<220>
<221> CDS
<222> (296)..(2158)

<400> 1
gagagctgcc tgctcagaca acagacacgc gaggtcagga agaagccgct tataaattac 60

cgcttccttc gcgccgccgc caacgccgag ccccgaggac cgcaagccca gaggacaagc 120

tgcgccaaga gggagtgcgg agcgttcacc cagcgggtca gagagcgagc gggcaggcag 180

cccccggccg gcggaacccg gcacagccga gcagagcgcg ggcggcgccg cagccacccc 240

agatccagaa ccagaaccac agcccttctg aggagctccc aaacaaagca aggag atg 298
Met

1

gcc acc aag gag aag ctg cag tgt ctg aaa gat ttc cac aag gac atc 346
Ala Thr Lys Glu Lys Leu Gln Cys Leu Lys Asp Phe His Lys Asp Ile
            5                   10                  15

ctg aag ccc tca cca ggg aag agc cca ggc acg cgg cct gag gac gag 394
Leu Lys Pro Ser Pro Gly Lys Ser Pro Gly Thr Arg Pro Glu Asp Glu
        20                  25                  30

gct gag gga aaa cct ccg cag agg gag aag tgg tct agc aag atc gac 442
Ala Glu Gly Lys Pro Pro Gln Arg Glu Lys Trp Ser Ser Lys Ile Asp
    35                  40                  45

ttt gtg ctc tct gtg gct ggc ggc ttc gtg ggc ttg ggc aac gtc tgg 490
Phe Val Leu Ser Val Ala Gly Gly Phe Val Gly Leu Gly Asn Val Trp
50                  55                  60                  65

cgc ttc ccg tac ctc tgc tac aag aat ggt gga ggt gcg ttt ctc ata 538
Arg Phe Pro Tyr Leu Cys Tyr Lys Asn Gly Gly Gly Ala Phe Leu Ile
                70                  75                  80

ccg tat ttt att ttc ctg ttt ggg agc ggc ctg cct gtg ttt ttc ttg 586
Pro Tyr Phe Ile Phe Leu Phe Gly Ser Gly Leu Pro Val Phe Phe Leu
            85                  90                  95

gag atc atc ata ggc cag tac acc tct gaa ggg ggc atc acc tgc tgg 634
Glu Ile Ile Ile Gly Gln Tyr Thr Ser Glu Gly Gly Ile Thr Cys Trp
        100                 105                 110

gaa aag atc tgc ccc ttg ttc tct ggt atc ggc tat gcc tcc gtt gta 682
Glu Lys Ile Cys Pro Leu Phe Ser Gly Ile Gly Tyr Ala Ser Val Val
    115                 120                 125

att gtg tcc ctc ctg aat gtc tac tac atc gtc atc ctg gcc tgg gcc 730
Ile Val Ser Leu Leu Asn Val Tyr Tyr Ile Val Ile Leu Ala Trp Ala

130                 135                 140                 145

aca tac tac ctg ttc cag tcc ttc cag aag gag ctg ccc tgg gca cac     778
Thr Tyr Tyr Leu Phe Gln Ser Phe Gln Lys Glu Leu Pro Trp Ala His
                150                 155                 160

tgc aac cac agc tgg aac aca cct cac tgc atg gag gac acc atg cgc     826
Cys Asn His Ser Trp Asn Thr Pro His Cys Met Glu Asp Thr Met Arg
            165                 170                 175

aag aac aag agt gtc tgg atc acc atc agc tcc acc aac ttc acc tcc     874
Lys Asn Lys Ser Val Trp Ile Thr Ile Ser Ser Thr Asn Phe Thr Ser
        180                 185                 190

cct gtc atc gag ttc tgg gag cgc aac gtg ctg agc ttg tcc cct gga     922
Pro Val Ile Glu Phe Trp Glu Arg Asn Val Leu Ser Leu Ser Pro Gly
    195                 200                 205

atc gac cac cca ggc tct ctg aaa tgg gac ctc gct ctc tgc ctt ctt     970
Ile Asp His Pro Gly Ser Leu Lys Trp Asp Leu Ala Leu Cys Leu Leu
210                 215                 220                 225

tta gtc tgg cta gtg tgt ttc ttc tgc atc tgg aag ggc gtc agg tcc     1018
Leu Val Trp Leu Val Cys Phe Phe Cys Ile Trp Lys Gly Val Arg Ser
                230                 235                 240

act ggg aag gtc gtc tac ttc aca gcc act ttt cca ttc gcc atg ctc     1066
Thr Gly Lys Val Val Tyr Phe Thr Ala Thr Phe Pro Phe Ala Met Leu
            245                 250                 255

ctg gtg ctg ctg gtc cga ggg ctg acg ctg ccg ggc gcg ggc gca ggc     1114
Leu Val Leu Leu Val Arg Gly Leu Thr Leu Pro Gly Ala Gly Ala Gly
        260                 265                 270

atc aag ttc tat ctg tat cct gac atc acc cgc ctt gag gac cca cag     1162
Ile Lys Phe Tyr Leu Tyr Pro Asp Ile Thr Arg Leu Glu Asp Pro Gln

275                 280                 285

gtg tgg att gac gct ggg act cag ata ttc ttc tct tat gcc atc tgc      1210
Val Trp Ile Asp Ala Gly Thr Gln Ile Phe Phe Ser Tyr Ala Ile Cys
290                 295                 300                 305

ctg ggg gct atg acc tcg ctg ggg agc tac aac aag tac aag tat aac      1258
Leu Gly Ala Met Thr Ser Leu Gly Ser Tyr Asn Lys Tyr Lys Tyr Asn
                310                 315                 320

tcg tac agg gac tgt atg ctg ctg gga tgc ctg aac agt ggt acc agt      1306
Ser Tyr Arg Asp Cys Met Leu Leu Gly Cys Leu Asn Ser Gly Thr Ser
            325                 330                 335

ttt gtg tct ggc ttc gca att ttt tcc atc ctg ggc ttc atg gca caa      1354
Phe Val Ser Gly Phe Ala Ile Phe Ser Ile Leu Gly Phe Met Ala Gln
        340                 345                 350

gag caa ggg gtg gac att gct gat gtg gct gag tca ggt cct ggc ctg      1402
Glu Gln Gly Val Asp Ile Ala Asp Val Ala Glu Ser Gly Pro Gly Leu
    355                 360                 365

gcc ttc att gcc tac cca aaa gct gtg aca atg atg ccg ctg ccc aca      1450
Ala Phe Ile Ala Tyr Pro Lys Ala Val Thr Met Met Pro Leu Pro Thr
370                 375                 380                 385

ttt tgg tcc att ctt ttt ttt att atg ctt ctc ttg ctt gga ctg gat      1498
Phe Trp Ser Ile Leu Phe Phe Ile Met Leu Leu Leu Leu Gly Leu Asp
                390                 395                 400

agc cag ttt gtt gaa gtt gaa gga cag atc aca tcc ttg gtt gat ctt      1546
Ser Gln Phe Val Glu Val Glu Gly Gln Ile Thr Ser Leu Val Asp Leu
            405                 410                 415

tac cca tcc ttc cta agg aag ggt tat cgt cgg gaa atc ttc atc gcc      1594
Tyr Pro Ser Phe Leu Arg Lys Gly Tyr Arg Arg Glu Ile Phe Ile Ala

        420                 425                 430

ttc gtg tgt agc atc agc tac ctg ctg ggg ctg acg atg gtg acg gag 1642
Phe Val Cys Ser Ile Ser Tyr Leu Leu Gly Leu Thr Met Val Thr Glu
    435                 440                 445

ggt ggc atg tat gtg ttt cag ctc ttt gac tac tat gca gct agc ggt 1690
Gly Gly Met Tyr Val Phe Gln Leu Phe Asp Tyr Tyr Ala Ala Ser Gly
450                 455                 460                 465

gta tgc ctt ttg tgg gtt gca ttc ttt gaa tgt ttt gtt att gcc tgg 1738
Val Cys Leu Leu Trp Val Ala Phe Phe Glu Cys Phe Val Ile Ala Trp
                470                 475                 480

ata tat gga ggt gat aac ctt tat gat ggt att gag gac atg att ggc 1786
Ile Tyr Gly Gly Asp Asn Leu Tyr Asp Gly Ile Glu Asp Met Ile Gly
            485                 490                 495

tat cgg ccc ggg ccc tgg atg aag tac agc tgg gct gtg atc act cca 1834
Tyr Arg Pro Gly Pro Trp Met Lys Tyr Ser Trp Ala Val Ile Thr Pro
        500                 505                 510

gtt ctc tgt gtt gga tgt ttc atc ttc tcg ctc gtc aag tac gta ccc 1882
Val Leu Cys Val Gly Cys Phe Ile Phe Ser Leu Val Lys Tyr Val Pro
    515                 520                 525

ctg acc tac aac aaa aca tac gtg tac ccc aac tgg gcc att ggg ctg 1930
Leu Thr Tyr Asn Lys Thr Tyr Val Tyr Pro Asn Trp Ala Ile Gly Leu
530                 535                 540                 545

ggc tgg agc ctg gcc ctt tcc tcc atg ctc tgc gtt ccc ttg gtc atc 1978
Gly Trp Ser Leu Ala Leu Ser Ser Met Leu Cys Val Pro Leu Val Ile
                550                 555                 560

gtc atc cgc ctc tgc cag act gag ggg ccg ttc ctt gtg aga gtc aag 2026
Val Ile Arg Leu Cys Gln Thr Glu Gly Pro Phe Leu Val Arg Val Lys

            565                 570                 575

tac ctg ctg acc cca agg gaa ccc aac cgc tgg gct gtg gag cgc gag 2074
Tyr Leu Leu Thr Pro Arg Glu Pro Asn Arg Trp Ala Val Glu Arg Glu
        580                 585                 590

gga gcc aca cct tac aac tct cgc acc gtc atg aac ggc gct ctc gtg 2122
Gly Ala Thr Pro Tyr Asn Ser Arg Thr Val Met Asn Gly Ala Leu Val
    595                 600                 605

aaa ccg acc cac atc att gtg gag acc atg atg tga gctctctcgg 2168
Lys Pro Thr His Ile Ile Val Glu Thr Met Met
610                 615                 620

gtcgacgggg ccggcggctt tcctgctgtt tactaacatt agattctcat aggaccaggt 2228

ttacagagct ttatatttgc actaggattt tttttttttt gtaattgtca cagaaaatgt 2288

aattgtgggt atgtgtgcgt gcgtgtgtgt gtgtgtgtgt atcgtgtgtg tgtgttttgt 2348

tttgatttgg gggatatttt gtacaaaaag aaaacccacg ggaagatgtc cgtggagagg 2408

cagagctttc atactgaatt agatgtattt tatgggaatt tggtaaattt ttctttgtat 2468

tttttttttt acatataagt atatatacac ttagagattg tcatatactt ttaccacttg 2528

aattgatctt cttgccagca atagatctca ttttcaaaag caattcttcg gtgctgtgta 2588

gctggcagaa agttctgtcc agtaaacgca ggatggaatt ttcctgggac tctacaccca 2648

tcttaaggtg gtataccttc caaatcctgg ttcagatgga agaaatagca ggagagagga 2708

cccattagct ggcagaccca gggggaagaa aggagggctg tgaggagata cctcattaaa 2768

cttggcttag tgaagaagag agatgccaaa ggaatgaacc aacccttcac ataaaggaga 2828

ctggctgaag ctgaatgagg aggccctata gcagaagtct gattctaaga gcagtagaaa 2888

cttgtaccag aagcaaaatc ccacttttaa ttttgagatg gtgagtggat agtcagtaga 2948

ccgtcagaac cactggccag agagggagct gctagagatc caagaaggct ggcaggagtg 3008

aggctcacaa ctcagcctcg caagaggtgg cagaggcaca ggaggccaca gtccttcctg 3068

gggcattcca ggcagagaag gagcagaggc tctcccggca ggagctgggg tctcagggct 3128

cagatgagtc tgttgcattt gaatggggtc atagcaggtt ctggtcattc cccaagcaac 3188

atctcagcat ctcttaaagt tgcctgcagg aatgaagcat gacatacctg ttgagggact 3248

aggggagtgg tggggaggtg agtggaccaa aggatatagg ccccaggcat gcagatgggc 3308

ccggtgtcgg ggaggggtgc tttctttcct catctcccca ctccccactc tcagcctggg 3368

agactcctgc caagccctca ttaaagatgc caccctgggc tgccctggca cctagcaagg 3428

cacaccaaga acagcttttg agtctgtatc ctccactggg ggaagtgctc ccagttcaga 3488

acaagggcag cccgtggtgc tgacctagga tataacaaag ctcttcactt caaaacccct 3548

gcaatagctg ggtttacaga catttaccac ctgcggaccc aaaagagaag gcctaggaga 3608

gttttctaga aggttgggat tgtcagggtc ctggcccctc agaactggct tgatcaaggg 3668

ccttatgtgg agcagaggtt gtctctgaac caggagagaa ggtactatac ctttcaaatc 3728

cccagggcag acacaccccc acccagcccc tatttggacc taaactgtgc catttgaaca 3788

gtcacttcca agctcagtct aaatgaaacc gaaacgtgac cacgcacaaa ggcagtcact 3848

gcctcgaggg gtgcagaccg cagaattttc acagcagggg ctcttggaac cctggaaacc 3908

cccttcttaa atttgggagg aggagtatgc ctttggtgtc cccctcccaa ggggcaattc 3968

tgaaccccat ctttggcagg catacatatt tcactgtttc caaagctatc tactctgcca 4028

aacaacaccc agtcctattc caaactctca acgattctat cttgttcctg tttttctatg 4088

tatttatggt tgccgtttgt gtctgatttg attttactgt tttttccctg attttatgga 4148

gtagcattgt gacctgtttt cctttgtctt atataacttt agtaaactaa ccactgtcaa 4208

tgattgaggg caggtggcac gtggggaaga ggggacttgg cacgcagtgg ctacctgggc 4268

atttgtggtc atttcagttt ccatctcccc agcgggggct ccctgggtga aaggccacag 4328

tattttgggt tggtaggcaa attgcaacat tctggacatg gcctgaggaa ggcctcttct 4388

tataagattc tcagaccaaa ttctagacca aagacacagg cagaccaagt ccccaggccc 4448

cgcctggaag gaagtcgttc ctcaactctc cccaaggcac ctgtctccaa tcagagccct 4508

ctcgcccagc cagccctggc tctgtgtgca gagcatagct ctgcgagtac ctgtgtaata 4568

atgctcaacc ttcatgtctc cgtataaacg aaactttcca tgagagctca tgactctggt 4628

ccacctgtct atagagaatg ggcaaagtcc ttcacctgct ttctgcttgg gatgggtcag 4688

aaatgctgat gcccgcacat agcccagcca gccagatctg gaaaggaagc gagggggttg 4748

tttaaatcaa ttttttaaga tgaagaagtg ggagacactg cgttgagatg ggccatgcta 4808

gggccacaga gatttcctga cggtcaggga gagaagggcc tccagggtcc cctaacccaa 4868

cgcccttgtt gtaaatgagg taactgaggc tcagggaggc actgtgagcc aggaatggat 4928

tttcttgaaa cagctctagc tgcaggttct ccgaggtagg tgcagggaat ggtgagtgtc 4988

taaccagggc tacatccagc aacatcctca aggtcttcct gacaaccaaa gacaagcctt 5048

tatggaaaag gaaatgcgct cccctccatg ttcagggatg aggggagcag cagcagccac 5108

actcccacca tcctcacaga attcctggac ccatgcggtg gctccgtgag ctgggtgact 5168

ccagcctcac ctgcacaccc cagccctgca cggggccctc cttcctccca gcagcccttg 5228

gtgagctagg aattgagatc cctgtttgtg aaagagggaa ctgaggtgca gagaagccag 5288

aggtgtgcca gatccttagg caggatttag atgaagtcgc cctggctcca gactgacccc 5348

gaggctctgc ggggagtttc caggcagcag gaagtggcct tggatgctct ccttccagga 5408

cagcataacc cctgggccat gtgcagctcc ttcactgccc cctggatccc cagcataccc 5468

ccaaagacag tggggaaaca caaggggaga gcacagcatg gcccctccag cccacttcag 5528

ggcactcttg tatcacccgg gtaccgccac actggtcccc cacccagcca gcatctccca 5588

gcacagcccc tctccctggg gaaatgctct gggtagccag tctaaaggca gaggcaccta 5648

actgctcccc gcagcccacc ccacccaaga ttcagacaca agccaggaaa ggacccaaga 5708

gaaaatcctt caaggtggcc tgaggtccca tccctccctc agacccatgt ggtcccaggc 5768

caggctgcct gggacacggt aaataccact gtgtgcaaaa atcgaagtac aaaaccacaa 5828

gactaaacaa aacaaaccca gagagccaaa cttgtagagg tgggcagtcc agaaagcagg 5888

gggcagccct ccccctttcc ttctctccct gatcctcaga atatatattg ttgtaatagg 5948

aagcattttt gcattgttct cttgtgggtg tcactacaga catgttctgg cgtgttctcc 6008

gagggatgga gcatcctgtt atatatttga cttcaaattg agatgttggc ttcatttttt 6068

ttttttaccc aattaatctc ccaatcccta gcaactgtga ctctgtattt agcacaagag 6128

aaagctgaga atgtgggtct tgcctccttc cagaaatatg tctggctcat caggacattt 6188

ttttaaaact tcaaaatatt tttaagatat tttaaacttt tataaaaaaa aaatcaacca 6248

acaagagact tttctgagga ggaacatttg tatttgaaca agatccttgg tgtgtagttc 6308

agtcttgcag tatacaagct tttgtgtata aatgttttat gatatgattc cctgtatttt 6368

gcaggggttt ttttctcttt tgctttttag ataaatatgt atatcaatat tttaaattca 6428

tctttgcttt ttttagagga gtttgtaatc accttataac atgaaaataa acatttcctt 6488

tttaacatcc aaaaaaaaaa aaaaaaaa 6516

<210> 2
<211> 620
<212> PRT
<213> Homo sapiens

<400> 2

Met Ala Thr Lys Glu Lys Leu Gln Cys Leu Lys Asp Phe His Lys Asp
1               5                   10                  15

Ile Leu Lys Pro Ser Pro Gly Lys Ser Pro Gly Thr Arg Pro Glu Asp
            20                  25                  30

Glu Ala Glu Gly Lys Pro Pro Gln Arg Glu Lys Trp Ser Ser Lys Ile
        35                  40                  45

Asp Phe Val Leu Ser Val Ala Gly Gly Phe Val Gly Leu Gly Asn Val
    50                  55                  60

Trp Arg Phe Pro Tyr Leu Cys Tyr Lys Asn Gly Gly Gly Ala Phe Leu
65                  70                  75                  80

Ile Pro Tyr Phe Ile Phe Leu Phe Gly Ser Gly Leu Pro Val Phe Phe
                85                  90                  95

Leu Glu Ile Ile Ile Gly Gln Tyr Thr Ser Glu Gly Gly Ile Thr Cys
            100                 105                 110

Trp Glu Lys Ile Cys Pro Leu Phe Ser Gly Ile Gly Tyr Ala Ser Val
        115                 120                 125

Val Ile Val Ser Leu Leu Asn Val Tyr Tyr Ile Val Ile Leu Ala Trp
    130                 135                 140

Ala Thr Tyr Tyr Leu Phe Gln Ser Phe Gln Lys Glu Leu Pro Trp Ala
145                 150                 155                 160

His Cys Asn His Ser Trp Asn Thr Pro His Cys Met Glu Asp Thr Met
                165                 170                 175

Arg Lys Asn Lys Ser Val Trp Ile Thr Ile Ser Ser Thr Asn Phe Thr
            180                 185                 190

Ser Pro Val Ile Glu Phe Trp Glu Arg Asn Val Leu Ser Leu Ser Pro
        195                 200                 205

Gly Ile Asp His Pro Gly Ser Leu Lys Trp Asp Leu Ala Leu Cys Leu
    210                 215                 220

Leu Leu Val Trp Leu Val Cys Phe Phe Cys Ile Trp Lys Gly Val Arg
225                 230                 235                 240

Ser Thr Gly Lys Val Val Tyr Phe Thr Ala Thr Phe Pro Phe Ala Met
                245                 250                 255

Leu Leu Val Leu Leu Val Arg Gly Leu Thr Leu Pro Gly Ala Gly Ala
            260                 265                 270

Gly Ile Lys Phe Tyr Leu Tyr Pro Asp Ile Thr Arg Leu Glu Asp Pro
        275                 280                 285

Gln Val Trp Ile Asp Ala Gly Thr Gln Ile Phe Phe Ser Tyr Ala Ile
    290                 295                 300

Cys Leu Gly Ala Met Thr Ser Leu Gly Ser Tyr Asn Lys Tyr Lys Tyr
305                 310                 315                 320

Asn Ser Tyr Arg Asp Cys Met Leu Leu Gly Cys Leu Asn Ser Gly Thr
                325                 330                 335

Ser Phe Val Ser Gly Phe Ala Ile Phe Ser Ile Leu Gly Phe Met Ala
            340                 345                 350

Gln Glu Gln Gly Val Asp Ile Ala Asp Val Ala Glu Ser Gly Pro Gly
        355                 360                 365

Leu Ala Phe Ile Ala Tyr Pro Lys Ala Val Thr Met Met Pro Leu Pro
    370                 375                 380

Thr Phe Trp Ser Ile Leu Phe Phe Ile Met Leu Leu Leu Leu Gly Leu
385                 390                 395                 400

Asp Ser Gln Phe Val Glu Val Glu Gly Gln Ile Thr Ser Leu Val Asp
                405                 410                 415

Leu Tyr Pro Ser Phe Leu Arg Lys Gly Tyr Arg Arg Glu Ile Phe Ile
            420                 425                 430

Ala Phe Val Cys Ser Ile Ser Tyr Leu Leu Gly Leu Thr Met Val Thr
        435                 440                 445

Glu Gly Gly Met Tyr Val Phe Gln Leu Phe Asp Tyr Tyr Ala Ala Ser
    450                 455                 460

Gly Val Cys Leu Leu Trp Val Ala Phe Phe Glu Cys Phe Val Ile Ala
465                 470                 475                 480

Trp Ile Tyr Gly Gly Asp Asn Leu Tyr Asp Gly Ile Glu Asp Met Ile
                485                 490                 495

Gly Tyr Arg Pro Gly Pro Trp Met Lys Tyr Ser Trp Ala Val Ile Thr
            500                 505                 510

Pro Val Leu Cys Val Gly Cys Phe Ile Phe Ser Leu Val Lys Tyr Val
        515                 520                 525

Pro Leu Thr Tyr Asn Lys Thr Tyr Val Tyr Pro Asn Trp Ala Ile Gly
    530                 535                 540

Leu Gly Trp Ser Leu Ala Leu Ser Ser Met Leu Cys Val Pro Leu Val
545                 550                 555                 560

Ile Val Ile Arg Leu Cys Gln Thr Glu Gly Pro Phe Leu Val Arg Val
                565                 570                 575

Lys Tyr Leu Leu Thr Pro Arg Glu Pro Asn Arg Trp Ala Val Glu Arg
            580                 585                 590

Glu Gly Ala Thr Pro Tyr Asn Ser Arg Thr Val Met Asn Gly Ala Leu
        595                 600                 605

Val Lys Pro Thr His Ile Ile Val Glu Thr Met Met
    610                 615                 620

<210> 3
<211> 1286
<212> DNA
<213> Homo sapiens

<220>
<221> CDS
<222> (296)..(898)

<400> 3
gagagctgcc tgctcagaca acagacacgc gaggtcagga agaagccgct tataaattac 60

cgcttccttc gcgccgccgc caacgccgag ccccgaggac cgcaagccca gaggacaagc 120

tgcgccaaga gggagtgcgg agcgttcacc cagcgggtca gagagcgagc gggcaggcag 180

cccccggccg gcggaacccg gcacagccga gcagagcgcg ggcggcgccg cagccacccc 240

agatccagaa ccagaaccac agcccttctg aggagctccc aaacaaagca aggag atg 298
Met
1

gcc acc aag gag aag ctg cag tgt ctg aaa gat ttc cac aag gac atc 346
Ala Thr Lys Glu Lys Leu Gln Cys Leu Lys Asp Phe His Lys Asp Ile
            5                   10                  15

ctg aag ccc tca cca ggg aag agc cca ggc acg cgg cct gag gac gag 394
Leu Lys Pro Ser Pro Gly Lys Ser Pro Gly Thr Arg Pro Glu Asp Glu
        20                  25                  30

gct gag gga aaa cct ccg cag agg gag aag tgg tct agc aag atc gac 442
Ala Glu Gly Lys Pro Pro Gln Arg Glu Lys Trp Ser Ser Lys Ile Asp
    35                  40                  45

ttt gtg ctc tct gtg gct ggc ggc ttc gtg ggc ttg ggc aac gtc tgg 490
Phe Val Leu Ser Val Ala Gly Gly Phe Val Gly Leu Gly Asn Val Trp

50                  55                  60                  65

cgc ttc ccg tac ctc tgc tac aag aat ggt gga ggt gcg ttt ctc ata     538
Arg Phe Pro Tyr Leu Cys Tyr Lys Asn Gly Gly Gly Ala Phe Leu Ile
                70                  75                  80

ccg tat ttt att ttc ctg ttt ggg agc ggc ctg cct gtg ttt ttc ttg     586
Pro Tyr Phe Ile Phe Leu Phe Gly Ser Gly Leu Pro Val Phe Phe Leu
            85                  90                  95

gag atc atc ata ggc cag tac acc tct gaa ggg ggc atc acc tgc tgg     634
Glu Ile Ile Ile Gly Gln Tyr Thr Ser Glu Gly Gly Ile Thr Cys Trp
        100                 105                 110

gaa aag atc tgc ccc ttg ttc tct ggt atc ggc tat gcc tcc gtt gta     682
Glu Lys Ile Cys Pro Leu Phe Ser Gly Ile Gly Tyr Ala Ser Val Val
    115                 120                 125

att gtg tcc ctc ctg aat gtc tac tac atc gtc atc ctg gcc tgg gcc     730
Ile Val Ser Leu Leu Asn Val Tyr Tyr Ile Val Ile Leu Ala Trp Ala
130                 135                 140                 145

aca tac tac ctg ttc cag tcc ttc cag aag gag ctg ccc tgg gca cac     778
Thr Tyr Tyr Leu Phe Gln Ser Phe Gln Lys Glu Leu Pro Trp Ala His
                150                 155                 160

tgc aac cac agc tgg aac aca cct cac tgc atg gag gac acc atg cgc     826
Cys Asn His Ser Trp Asn Thr Pro His Cys Met Glu Asp Thr Met Arg
            165                 170                 175

aag aac aag agt gtc tgg atc acc atc agc tcc acc aac ttc acc tcc     874
Lys Asn Lys Ser Val Trp Ile Thr Ile Ser Ser Thr Asn Phe Thr Ser
        180                 185                 190

cct gtc atc gag ttc tgg gag taa ggccacctca ttgaagcaag gaggaggaca     928
Pro Val Ile Glu Phe Trp Glu

195                 200

tgggtggggc agagaggatg gcccacttcc ttatctcctc ccctgggata caggagccac    988

tgtcttcggg ggagtgggag gagggtgcag atctggagat aaatttatgc ctttggccat    1048

agacaggact ggaatcccag ttctgccact tactggctgt ttgaccttag gcaagccact    1108

cctcctttct gagcctcagt ttctttacct gtaagataaa aagaatgaat cattactacc    1168

ctgtgaggca ggtatgcata cttatgttgt ctaaaaatga tacagttttc ttaatgtatt    1228

ttaggaaaaa atatattctg ttattgcaca gagaaaaaaa aaaaaaaaaa aaaaaaaa    1286

<210>  4
<211>  200
<212>  PRT
<213>  Homo sapiens

<400>  4

Met Ala Thr Lys Glu Lys Leu Gln Cys Leu Lys Asp Phe His Lys Asp
1               5                   10                  15

Ile Leu Lys Pro Ser Pro Gly Lys Ser Pro Gly Thr Arg Pro Glu Asp
            20                  25                  30

Glu Ala Glu Gly Lys Pro Pro Gln Arg Glu Lys Trp Ser Ser Lys Ile
        35                  40                  45

Asp Phe Val Leu Ser Val Ala Gly Gly Phe Val Gly Leu Gly Asn Val
    50                  55                  60

Trp Arg Phe Pro Tyr Leu Cys Tyr Lys Asn Gly Gly Gly Ala Phe Leu
65                  70                  75                  80

Ile Pro Tyr Phe Ile Phe Leu Phe Gly Ser Gly Leu Pro Val Phe Phe
                85                  90                  95

Leu Glu Ile Ile Ile Gly Gln Tyr Thr Ser Glu Gly Gly Ile Thr Cys
            100                 105                 110

Trp Glu Lys Ile Cys Pro Leu Phe Ser Gly Ile Gly Tyr Ala Ser Val
        115                 120                 125

Val Ile Val Ser Leu Leu Asn Val Tyr Tyr Ile Val Ile Leu Ala Trp
    130                 135                 140

Ala Thr Tyr Tyr Leu Phe Gln Ser Phe Gln Lys Glu Leu Pro Trp Ala
145                 150                 155                 160

His Cys Asn His Ser Trp Asn Thr Pro His Cys Met Glu Asp Thr Met
                165                 170                 175

Arg Lys Asn Lys Ser Val Trp Ile Thr Ile Ser Ser Thr Asn Phe Thr
            180                 185                 190

Ser Pro Val Ile Glu Phe Trp Glu
        195                 200

<210> 5
<211> 6381
<212> DNA
<213> Homo sapiens

<220>
<221> CDS
<222> (304)..(1383)

<400> 5
gagagctgcc tgctcagaca acagacacgc gaggtcagga agaagccgct tataaattac 60

cgcttccttc gcgccgccgc caacgccgag ccccgaggac cgcaagccca gaggacaagc 120

tgcgccaaga gggagtgctg gagctgttca cccagcgggt cagagagcga gccgggcagg 180

ccagcccccg gccggcggaa ccctggcaca gcctgagcag atgctgcggg cggcgccgca 240

gccaccccag atccagaacc agaaccacag cccttctgag gagctcccaa acaaagcaag 300

gag atg gcc acc aag gag aag ctg cag tgt ctg aaa gat ttc cac aag 348
    
    1               5                   10                  15

gac atc ctg aag ccc tca cca ggg aag agc cca ggc acg cgg cct gag 396

                20                  25                  30

gac gag gct gag gga aaa cct ccg cag agg gag aag tgg tct agc aag 444

            35                  40                  45

atc gac ttt gtg ctc tct gtg gct ggc ggc ttc gtg ggc ttg ggc aac 492


        50                  55                  60

gtc tgg cgc ttc ccg tac ctc tgc tac aag aat ggt gga ggt gcg ttt 540

    65                  70                  75

ctc ata ccg tat ttt att ttc ctg ttt ggg agc ggc ctg cct gtg ttt 588

80                  85                  90                  95

ttc ttg gag atc atc ata ggc cag tac acc tct gaa ggg ggc atc acc 636

                100                 105                 110

tgc tgg gaa aag atc tgc ccc ttg ttc tct ggt atc ggc tat gcc tcc 684

            115                 120                 125

gtt gta att gtg tcc ctc ctg aat gtc tac tac atc gtc atc ctg gcc 732

        130                 135                 140

tgg gcc aca tac tac ctg ttc cag tcc ttc cag aag gag ctg ccc tgg 780

    145                 150                 155

gca cac tgc aac cac agc tgg aac aca cct cac tgc atg gag gac acc 828

160                 165                 170                 175

atg cgc aag aac aag agt gtc tgg atc acc atc agc tcc acc aac ttc 876

                180                 185                 190

acc tcc cct gtc atc gag ttc tgg gag cgc aac gtg ctg agc ttg tcc 924


            195                 200                 205

cct gga atc gac cac cca ggc tct ctg aaa tgg gac ctc gct ctc tgc 972

        210                 215                 220

ctt ctt tta gtc tgg cta gtg tgt ttc ttc tgc atc tgg aag ggc gtc 1020

    225                 230                 235

agg tcc act ggg aag gtc gtc tac ttc aca gcc act ttt cca ttc gcc 1068

240                 245                 250                 255

atg ctc ctg gtg ctg ctg gtc cga ggg ctg acg ctg ccg ggc gcg ggc 1116

                260                 265                 270

gca ggc atc aag ttc tat ctg tat cct gac atc acc cgc ctt gag gac 1164

            275                 280                 285

cca cag gtg tgg att gac gct ggg act cag ata ttc ttc tct tat gcc 1212

        290                 295                 300

atc tgc ctg ggg gct atg acc tcg ctg ggg agc tac aac aag tac aag 1260

    305                 310                 315

tat aac tcg tac agg tcc tgg cct ggc ctt cat tgc cta ccc aaa agc 1308
Tyr Asn Ser Tyr Arg Ser Trp Pro Gly Leu His Cys Leu Pro Lys Ser
320                 325                 330                 335

tgt gac aat gat gcc gct gcc cac att ttg gtc cat tct ttt ttt tat 1356
Cys Asp Asn Asp Ala Ala Ala His Ile Leu Val His Ser Phe Phe Tyr

                340                 345                 350

tat gct tct ctt gct tgg act gga tag ccagtttgtt gaagttgaag 1403
Tyr Ala Ser Leu Ala Trp Thr Gly
            355

gacagatcac atccttggtt gatctttacc catccttcct aaggaagggt tatcgtcggg 1463

aaatcttcat cgccttcgtg tgtagcatca gctacctgct ggggctgacg atggtgacgg 1523

agggtggcat gtatgtgttt cagctctttg actactatgc agctagcggt gtatgccttt 1583

tgtgggttgc attctttgaa tgttttgtta ttgcctggat atatggaggt gataaccttt 1643

atgatggtat tgaggacatg attggctatc ggcccgggcc ctggatgaag tacagctggg 1703

ctgtgatcac tccagttctc tgtgttggat gtttcatctt ctcgctcgtc aagtacgtac 1763

ccctgaccta caacaaaaca tacgtgtacc ccaactgggc cattgggctg ggctggagcc 1823

tggccctttc ctccatgctc tgcgttccct tggtcatcgt catccgcctc tgccagactg 1883

aggggccgtt ccttgtgaga gtcaagtacc tgctgacccc aagggaaccc aaccgctggg 1943

ctgtggagcg cgagggagcc acaccttaca actctcgcac cgtcatgaac ggcgctctcg 2003

tgaaaccgac ccacatcatt gtggagacca tgatgtgagc tctctcgggt cgacggggcc 2063

ggcggctttc ctgctgttta ctaacattag attctcatag gaccaggttt acagagcttt 2123

atatttgcac taggattttt ttttttttgt aattgtcaca gaaaatgtaa ttgtgggtat 2183

gtgtgcgtgc gtgtgtgtgt gtgtgtgtat cgtgtgtgtg tgttttgttt tgatttgggg 2243

gatattttgt acaaaaagaa aacccacggg aagatgtccg tggagaggca gagctttcat 2303

actgaattag atgtatttta tgggaatttg gtaaattttt ctttgtattt tttttttac 2363

atataagtat atatacactt agagattgtc atatactttt accacttgaa ttgatcttct 2423

tgccagcaat agatctcatt ttcaaaagca attcttcggt gctgtgtagc tggcagaaag 2483

ttctgtccag taaacgcagg atggaatttt cctgggactc tacacccatc ttaaggtggt 2543

ataccttcca aatcctggtt cagatggaag aaatagcagg agagaggacc cattagctgg 2603

cagacccagg gggaagaaag gagggctgtg aggagatacc tcattaaact tggcttagtg 2663

aagaagagag atgccaaagg aatgaaccaa cccttcacat aaaggagact ggctgaagct 2723

gaatgaggag gccctatagc agaagtctga ttctaagagc agtagaaact tgtaccagaa 2783

gcaaaatccc acttttaatt ttgagatggt gagtggatag tcagtagacc gtcagaacca 2843

ctggccagag agggagctgc tagagatcca agaaggctgg caggagtgag gctcacaact 2903

cagcctcgca agaggtggca gaggcacagg aggccacagt ccttcctggg gcattccagg 2963

cagagaagga gcagaggctc tcccggcagg agctggggtc tcagggctca gatgagtctg 3023

ttgcatttga atggggtcat agcaggttct ggtcattccc caagcaacat ctcagcatct 3083

cttaaagttg cctgcaggaa tgaagcatga catacctgtt gagggactag gggagtggtg 3143

gggaggtgag tggaccaaag gatataggcc ccaggcatgc agatgggccc ggtgtcgggg 3203

aggggtgctt tctttcctca tctccccact ccccactctc agcctgggag actcctgcca 3263

agccctcatt aaagatgcca ccctgggctg ccctggcacc tagcaaggca caccaagaac 3323

agcttttgag tctgtatcct ccactggggg aagtgctccc agttcagaac aagggcagcc 3383

cgtggtgctg acctaggata taacaaagct cttcacttca aaacccctgc aatagctggg 3443

tttacagaca tttaccacct gcggacccaa aagagaaggc ctaggagagt tttctagaag 3503

gttgggattg tcagggtcct ggcccctcag aactggcttg atcaagggcc ttatgtggag 3563

cagaggttgt ctctgaacca ggagagaagg tactatacct ttcaaatccc cagggcagac 3623

acacccccac ccagccccta tttggaccta aactgtgcca tttgaacagt cacttccaag 3683

ctcagtctaa atgaaaccga aacgtgacca cgcacaaagg cagtcactgc ctcgaggggt 3743

gcagaccgca gaattttcac agcaggggct cttggaaccc tggaaacccc cttcttaaat 3803

ttgggaggag gagtatgcct ttggtgtccc cctcccaagg ggcaattctg aaccccatct 3863

ttggcaggca tacatatttc actgtttcca aagctatcta ctctgccaaa caacacccag 3923

tcctattcca aactctcaac gattctatct tgttcctgtt tttctatgta tttatggttg 3983

ccgtttgtgt ctgatttgat tttactgttt tttccctgat tttatggagt agcattgtga 4043

cctgttttcc tttgtcttat ataactttag taaactaacc actgtcaatg attgagggca 4103

ggtggcacgt ggggaagagg ggacttggca cgcagtggct acctgggcat ttgtggtcat 4163

ttcagtttcc atctccccag cgggggctcc ctgggtgaaa ggccacagta ttttgggttg 4223

gtaggcaaat tgcaacattc tggacatggc ctgaggaagg cctcttctta taagattctc 4283

agaccaaatt ctagaccaaa gacacaggca gaccaagtcc ccaggccccg cctggaagga 4343

agtcgttcct caactctccc caaggcacct gtctccaatc agagccctct cgcccagcca 4403

gccctggctc tgtgtgcaga gcatagctct gcgagtacct gtgtaataat gctcaacctt 4463

catgtctccg tataaacgaa actttccatg agagctcatg actctggtcc acctgtctat 4523

agagaatggg caaagtcctt cacctgcttt ctgcttggga tgggtcagaa atgctgatgc 4583

ccgcacatag cccagccagc cagatctgga aaggaagcga gggggttgtt taaatcaatt 4643

ttttaagatg aagaagtggg agacactgcg ttgagatggg ccatgctagg gccacagaga 4703

tttcctgacg gtcagggaga gaagggcctc cagggtcccc taacccaacg cccttgttgt 4763

aaatgaggta actgaggctc agggaggcac tgtgagccag gaatggattt tcttgaaaca 4823

gctctagctg caggttctcc gaggtaggtg cagggaatgg tgagtgtcta accagggcta 4883

catccagcaa catcctcaag gtcttcctga caaccaaaga caagccttta tggaaaagga 4943

aatgcgctcc cctccatgtt cagggatgag ggagcagcag cagccacac tcccaccatc 5003

ctcacagaat tcctggaccc atgcggtggc tccgtgagct gggtgactcc agcctcacct 5063

gcacacccca gccctgcacg gggccctcct tcctcccagc agcccttggt gagctaggaa 5123

ttgagatccc tgtttgtgaa agagggaact gaggtgcaga gaagccagag gtgtgccaga 5183

tccttaggca ggatttagat gaagtcgccc tggctccaga ctgaccccga ggctctgcgg 5243

ggagtttcca ggcagcagga agtggccttg gatgctctcc ttccaggaca gcataacccc 5303

tgggccatgt gcagctcctt cactgccccc tggatcccca gcataccccc aaagacagtg 5363

gggaaacaca aggggagagc acagcatggc ccctccagcc cacttcaggg cactcttgta 5423

tcacccgggt accgccacac tggtccccca cccagccagc atctcccagc acagcccctc 5483

tccctgggga aatgctctgg gtagccagtc taaaggcaga ggcacctaac tgctccccgc 5543

agcccacccc acccaagatt cagacacaag ccaggaaagg acccaagaga aaatccttca 5603

aggtggcctg aggtcccatc cctccctcag acccatgtgg tcccaggcca ggctgcctgg 5663

gacacggtaa ataccactgt gtgcaaaaat cgaagtacaa aaccacaaga ctaaacaaaa 5723

caaacccaga gagccaaact tgtagaggtg ggcagtccag aaagcagggg gcagccctcc 5783

ccctttcctt ctctccctga tcctcagaat atatattgtt gtaataggaa gcatttttgc 5843

attgttctct tgtgggtgtc actacagaca tgttctggcg tgttctccga gggatggagc 5903

atcctgttat atatttgact tcaaattgag atgttggctt catttttttt ttttacccaa 5963

ttaatctccc aatccctagc aactgtgact ctgtatttag cacaagagaa agctgagaat 6023

gtgggtcttg cctccttcca gaaatatgtc tggctcatca ggacattttt ttaaaacttc 6083

aaaatatttt taagatattt taaactttta taaaaaaaaa atcaaccaac aagagacttt 6143

tctgaggagg aacatttgta tttgaacaag atccttggtg tgtagttcag tcttgcagta 6203

tacaagcttt tgtgtataaa tgttttatga tatgattccc tgtattttgc aggggttttt 6263

ttctcttttg ctttttagat aaatatgtat atcaatattt taaattcatc tttgcttttt 6323

ttagaggagt ttgtaatcac cttataacat gaaaataaac atttcctttt taacatcc 6381

<210> 6
<211> 359
<212> PRT
<213> Homo sapiens

<400> 6

Met Ala Thr Lys Glu Lys Leu Gln Cys Leu Lys Asp Phe His Lys Asp
1               5                   10                  15

Ile Leu Lys Pro Ser Pro Gly Lys Ser Pro Gly Thr Arg Pro Glu Asp
            20                  25                  30

Glu Ala Glu Gly Lys Pro Pro Gln Arg Glu Lys Trp Ser Ser Lys Ile
        35                  40                  45

Asp Phe Val Leu Ser Val Ala Gly Gly Phe Val Gly Leu Gly Asn Val
    50                  55                  60

Trp Arg Phe Pro Tyr Leu Cys Tyr Lys Asn Gly Gly Gly Ala Phe Leu
65                  70                  75                  80

Ile Pro Tyr Phe Ile Phe Leu Phe Gly Ser Gly Leu Pro Val Phe Phe
                85                  90                  95

Leu Glu Ile Ile Ile Gly Gln Tyr Thr Ser Glu Gly Gly Ile Thr Cys
            100                 105                 110

Trp Glu Lys Ile Cys Pro Leu Phe Ser Gly Ile Gly Tyr Ala Ser Val
        115                 120                 125

Val Ile Val Ser Leu Leu Asn Val Tyr Tyr Ile Val Ile Leu Ala Trp
    130                 135                 140

Ala Thr Tyr Tyr Leu Phe Gln Ser Phe Gln Lys Glu Leu Pro Trp Ala
145                 150                 155                 160

His Cys Asn His Ser Trp Asn Thr Pro His Cys Met Glu Asp Thr Met
                165                 170                 175

Arg Lys Asn Lys Ser Val Trp Ile Thr Ile Ser Ser Thr Asn Phe Thr
            180                 185                 190

Ser Pro Val Ile Glu Phe Trp Glu Arg Asn Val Leu Ser Leu Ser Pro
        195                 200                 205

Gly Ile Asp His Pro Gly Ser Leu Lys Trp Asp Leu Ala Leu Cys Leu
    210                 215                 220

Leu Leu Val Trp Leu Val Cys Phe Phe Cys Ile Trp Lys Gly Val Arg
225                 230                 235                 240

Ser Thr Gly Lys Val Val Tyr Phe Thr Ala Thr Phe Pro Phe Ala Met
                245                 250                 255

Leu Leu Val Leu Leu Val Arg Gly Leu Thr Leu Pro Gly Ala Gly Ala
            260                 265                 270

Gly Ile Lys Phe Tyr Leu Tyr Pro Asp Ile Thr Arg Leu Glu Asp Pro
        275                 280                 285

Gln Val Trp Ile Asp Ala Gly Thr Gln Ile Phe Phe Ser Tyr Ala Ile
    290                 295                 300

Cys Leu Gly Ala Met Thr Ser Leu Gly Ser Tyr Asn Lys Tyr Lys Tyr
305                 310                 315                 320

Asn Ser Tyr Arg Ser Trp Pro Gly Leu His Cys Leu Pro Lys Ser Cys
                325                 330                 335

Asp Asn Asp Ala Ala Ala His Ile Leu Val His Ser Phe Phe Tyr Tyr
            340                 345                 350

Ala Ser Leu Ala Trp Thr Gly
        355

<210>  7
<211>  222
<212>  DNA
<213>  Homo sapiens

<400>  7
gcctgggcca catactacct gttccagtcc ttccagaagg agctgccctg ggcacactgc      60

aaccacagct ggaacacacc tcactgcatg gaggacacca tgcgcaagaa caagagtgtc     120

tggatcacca tcagctccac caacttcacc tcccctgtca tcgagttctg ggagcgcaac     180

gtgctgagct tgtcccctgg aatcgaccac ccaggctctc tg                        222

<210>  8

<211>  74
<212>  PRT
<213>  Homo sapiens

<400>  8

Ala Trp Ala Thr Tyr Tyr Leu Phe Gln Ser Phe Gln Lys Glu Leu Pro
1               5                   10                  15

Trp Ala His Cys Asn His Ser Trp Asn Thr Pro His Cys Met Glu Asp
            20                  25                  30

Thr Met Arg Lys Asn Lys Ser Val Trp Ile Thr Ile Ser Ser Thr Asn
        35                  40                  45

Phe Thr Ser Pro Val Ile Glu Phe Trp Glu Arg Asn Val Leu Ser Leu
    50                  55                  60

Ser Pro Gly Ile Asp His Pro Gly Ser Leu
65                  70

<210>  9
<211>  31
<212>  DNA
<213>  Artificial

<220>
<223>  Synthetic DNA

<400>  9
aaaggatcca tggccaccaa ggagaagctg c                                    31

<210> 10
<211> 33
<212> DNA
<213> Artificial

<220>
<223> Synthetic DNA

<400> 10
aaatctagac atcatggtct ccacaatgat gtg 33

<210> 11
<211> 31
<212> DNA
<213> Artificial

<220>
<223> Synthetic DNA

<400> 11
ataggatccg gcctgggcca catatcacct g 31

<210> 12
<211> 32
<212> DNA
<213> Artificial

<220>
<223> Synthetic DNA

<400> 12
tatgaattcg ctttcagaga gcctgggtgg tc 32